# EUROPÄISCHE PATENTSCHRIFT

(11) **EP 0 595 164 B1**
(45) Veröffentlichungstag und Bekanntmachung des Hinweises auf die Patenterteilung: **27.12.1996**
(21) Anmeldenummer: 93116862.9
(22) Anmeldetag: 19.10.1993
(51) Int. Cl.: C07D 413/10, C07D 401/10, A61K 31/44

(54) **4-Heterocyclophenyl substituierte Dihydropyridine**
4-heterocyclephenyl substituted dihydropyridines
4-hétérocyclephényl dérivés de dihydropyridine

(30) Priorität: 30.10.1992 DE 4236706; 30.10.1992 DE 4236705
(43) Veröffentlichungstag der Anmeldung: 04.05.1994
(73) Patentinhaber: BAYER AG, 51368 Leverkusen (DE)
(72) Erfinder: Schohe-Loop, Rudolf, Dr., D-42113 Wuppertal (DE); Hartwig, Wolfgang, Dr., D-42113 Wuppertal (DE); Junge, Bodo, Dr., D-42399 Wuppertal (DE); Meier, Heinrich, Dr., D-42105 Wuppertal (DE); Gao, Zhan, Dr. , c/o Bayer Beijing L.Off.Unit 3009, Beijing 100020 Pr. (CN); Schmidt, Bernard, Dr., D-51789 Lindlar (DE); de Jonge, Maarten, Dr., D-51491 Overath (DE); Schuurman, Teunis, Dr., D-53797 Lohmar (DE)

(56) Entgegenhaltungen:
- EP-A- 0 373 645
- EP-A- 0 525 568
- US-A- 4 590 276
- TRENDS PHARMACOL. SCI. Bd. 11, Nr. 8, 1990, Seiten 309 - 310 'Nimodipine and the recovery of memory'

## Beschreibung

Die vorliegende Erfindung betrifft 4-Heterocyclylphenyl substituierte Dihydropyridine, Verfahren zu ihrer Herstellung und ihre Verwendung in Arzneimitteln, insbesondere als Mittel zur Behandlung von Erkrankungen des zentralen Nervensystems.

Die Verbindung Nimodipin und ihre cerebrale Wirksamkeit ist bekannt (vgl. DOS 28 15 578 und Trends Pharmacol. Sci., Bd. 11, Nr. 8, 1990, Seiten 309-310). Außerdem sind 4-Heterocyclylphenyl substituierte Dihydropyridine als Kalziumkanalblocker und Thromboxan A₂-Synthesehemmern und ihre blutdrucksenkende Wirkung bekannt (vgl. US 4 590 276, EP 373 645, JP 239 353, DE 38 16 361, EP 88 276, DE 32 39 273, DE 29 35 451, WO 84/02132, NE 68 038 17, US 3 488 359, US 3 574 843). Darüberhinaus sind auch N-alkylierte Dihydropyridindicarbonsäureester mit cerebraler Wirkung beschrieben (vgl. EP 525 568), die sich jedoch von den erfindungsgemäßen Wirkstoffen in zwei wesentlichen Strukturmerkmalen unterscheiden. Sie sind in 1-Position N-alkyliert und tragen am Phenylrest in 4-Position keinen ungesättigten Heterocyclrest.

Die durch die Maßgabe ausgeschlossene Verbindung ist aus J. Amer. Soc., Band 71, Seiten 4003-4007, (1949) bekannt.

Die vorliegende Erfindung betrifft 4-Heterocyclyl substituierte Dihydropyridine der allgemeinen Formel (I) 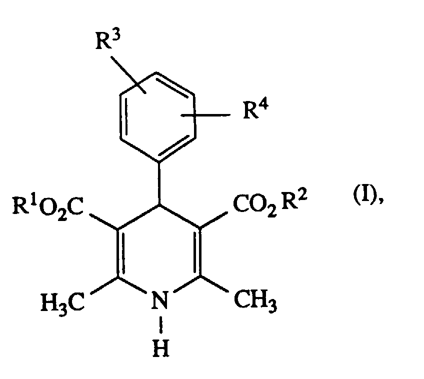 in welcher
- R¹ und R²: gleich oder verschieden sind und für Cycloalkyl mit 3 bis 8 Kohlenstoffatomen stehen, oder
für geradkettiges oder verzweigtes Alkyl mit bis zu 8 Kohlenstoffatomen stehen, das gegebenenfalls durch geradkettiges oder verzweigtes Alkoxy mit bis zu 6 Kohlenstoffatomen, Cyano, Cycloalkyl mit 3 bis 8 Kohlenstoffatomen oder Aryloxy mit 6 bis 10 Kohlenstoffatomen substituiert ist, das seinerseits bis zu 3-fach gleich oder verschieden durch Halogen, Cyano oder durch geradkettiges oder verzweigtes Alkyl oder Alkoxy mit bis zu 6 Kohlenstoffatomen substituiert sein kann,
- R³: für Wasserstoff, Halogen, Trifluormethyl oder Cyano steht,
und
- R⁴: für einen 5-gliedrigen ungesättigten, über ein Ring-Kohlenstoffatom angebundenen Heterocyclus mit bis zu 4 Heteroatomen aus der Reihe N und/oder O steht, der gegebenenfalls durch Hydroxy, geradkettiges oder verzweigtes Alkyl oder Alkoxycarbonyl mit jeweils bis zu 6 Kohlenstoffatomen, Trifluormethyl, Halogen, Carboxy oder durch eine Gruppe der Formel -NR⁵R⁶ substituiert ist,
oder
- R⁴: einen Rest der Formel 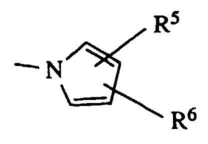 bedeutet,
worin
R⁵ und R⁶ gleich oder verschieden sind und Wasserstoff oder geradkettiges oder verzweigtes Alkyl oder Alkoxycarbonyl mit jeweils bis zu 4 Kohlenstoffatomen bedeuten,
und deren Salze,
mit der Maßgabe, daß wenn R¹ und R² für Ethyl und R³ für Wasserstoff stehen, R⁴ nicht den Rest der Formel 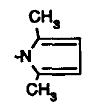 bedeuten darf.

Bevorzugt werden physiologisch unbedenkliche Salze der erfindungsgemäßen Verbindungen.

Physiologisch unbedenkliche Salze sind im allgemeinen Salze der erfindungsgemäßen Verbindungen mit anorganischen oder organischen Säuren. Bevorzugt werden Salze mit anorganischen Säuren wie beispielsweise Salzsäure, Bromwasserstoffsäure, Phosphorsäure oder Schwefelsäure, oder Salze mit organischen Carbon- oder Sulfonsäuren wie beispielsweise Essigsäure, Maleinsäure, Fumarsäure, Äpfelsäure, Zitronensäure, Weinsäure, Milchsäure, Benzoesäure, oder Methansulfonsäure, Ethansulfonsäure, Phenylsulfonsäure, Toluolsulfonsäure oder Naphthalindisulfonsäure.

Die erfindungsgemäßen Verbindungen existieren in stereoisomeren Formen, die sich entweder wie Bild und Spiegelbild (Enantiomere), oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen (vgl. E.L. Eliel, Stereochemistry of Carbon Compounds, McGraw Hill, 1962).

Ein 5-gliedriger ungestättigter Heterocyclus mit bis zu 4 Heteroatomen aus der Reihe N und/oder O steht im allgemeinen für Imidazolyl, Pyrrolyl, Pyrazolyl, Isoxazolyl, Furyl, Tetrazolyl, Oxazolyl, 1,2,4- und 1,3,4-Oxadiazolyl, 1,2,3-Triazolyl. Bevorzugt sind Tetrazolyl, 1,2,4-Oxadiazolyl und 1,3,4-Oxadiazolyl.

Bevorzugt sind Verbindungen der allgemeinen Formel (I),
in welcher
- R¹ und R²: gleich oder verschieden sind und für Cyclopropyl, Cyclopentyl, Cyclohexyl oder Cycloheptyl stehen, oder
für geradkettiges oder verzweigtes Alkyl mit bis zu 6 Kohlenstoffatomen stehen, das gegebenenfalls durch geradkettiges oder verzweigtes Alkoxy mit bis zu 4 Kohlenstoffatomen, Cyano, Cyclopropyl, Cyclopentyl, Cyclohexyl, Cycloheptyl oder Phenoxy substituiert ist, das seinerseits bis zu 3-fach gleich oder verschieden durch Fluor, Cyano oder durch geradkettiges oder verzweigtes Alkyl oder Alkoxy mit jeweils bis zu 4 Kohlenstoffatomen substituiert sein kann,
- R³: für Wasserstoff, Fluor, Chlor, Brom, Trifluormethyl oder Cyano steht,
und
- R⁴: für über ein Ring-Kohlenstoffatom gebundenes Tetrazolyl, 1,2,4-Oxadiazolyl oder 1,3,4-Oxadiazolyl steht, das gegebenenfalls durch Hydroxy, geradkettiges
oder verzweigtes Alkyl oder Alkoxycarbonyl mit jeweils bis zu 4 Kohlenstoffatomen, Trifluormethyl, Fluor, Chlor oder durch eine Gruppe der Formel -NR⁵R⁶ substituiert ist,
oder
- R⁴: einen Rest der Formel 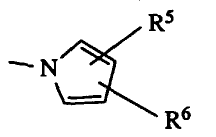 bedeutet,
worin
R⁵ und R⁶ gleich oder verschieden sind und Wasserstoff oder geradkettiges oder verzweigtes Alkyl oder Alkoxycarbonyl mit jeweils bis zu 3 Kohlenstoffatomen bedeuten.
und deren Salze,
mit der Maßgabe, daß wenn R¹ und R² für Ethyl und R³ für Wasserstoff stehen, R⁴ nicht den Rest der Formel 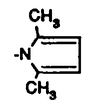 bedeuten darf.

Besonders bevorzugt sind Verbindungen der allgemeinen Formel (I),
in welcher
- R¹ und R²: gleich oder verschieden sind und für Cyclopentyl, Cyclohexyl oder Cycloheptyl stehen, oder
für geradkettiges oder verzweigtes Alkyl mit bis zu 4 Kohlenstoffatomen stehen, das gegebenenfalls durch Methoxy, Cyclopentyl, Cyclohexyl oder Cycloheptyl substituiert ist
- R³: für Wasserstoff, Chlor, Trifluormethyl oder Cyano steht,
und
- R⁴: für über ein Ring-Kohlenstoffatom gebundenes Tetrazolyl, 1,2,4-Oxadiazolyl oder 1,3,4-Oxadiazolyl steht, das gegebenenfalls durch geradkettiges oder verzweigtes Alkyl oder Alkoxycarbonyl mit jeweils bis zu 3 Kohlenstoffatomen, Trifluormethyl, Fluor, Chlor oder Amino substituiert ist,
oder
- R⁴: einen Rest der Formel 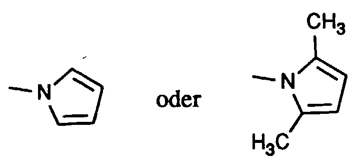 bedeutet.
und deren Salze,
mit der Maßgabe, daß wenn R¹ und R² für Ethyl und R³ für Wasserstoff stehen, R⁴ nicht den Rest der Formel 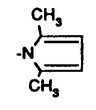 bedeuten darf.

Ganz besonders bevorzugt sind folgende Verbindungen der allgemeinen Formel (I), in welcher R⁴ für ein Ring-Kohlenstoff gebundenes Tetrazolyl, 1,2,4-Oxadiazolyl oder 1,3,4-Oxadiazolyl steht,
1,4-Dihydro-2,6-dimethyl-4-[3-(1,2,4-oxadiazol-3-yl)phenyl]-pyridin-3,5-dicarbonsäure-isopropyl-2-methoxyethyl-ester
4-[3-(5-Ethoxycarbonyl-1,2,4-oxadiazol-3-yl)-phenyl]-1,4-dihydro-2,6-dimethyl-pyridin-3-5-dicarbonsäure-isopropyl-2-methoxyethyl-ester
(+)-4-[3-(5-Ethoxycarbonyl-1,2,4-oxadiazol-3-yl)-phenyl]-1,4-dihydro-2,6-dimethyl-pyridin-3-5-dicarbonsäure-isopropyl-2-methoxyethyl-ester
(-)4-[3-(5-Ethoxycarbonyl-1,2,4-oxadiazol-3-yl)-phenyl]-1,4-dihydro-2,6-dimethyl-pyridin-3-5-dicarbonsäure-isopropyl-2-methoxyethyl-ester
1,4-Dihydro-2,6-dimethyl-4-[3-(tetrazol-5-yl)phenyl]-pyridin-3,5-dicarbonsäure-isopropyl-2-methoxyethyl-ester
1,4-Dihydro-2,6-dimethyl-4-[3-(1,3,4-oxadiazol-2-yl)-phenyl]-pyridin-3,5-dicarbonsäure-isopropyl-2-methoxyethyl-ester
(+)-1,4-Dihydro-2,6-dimethyl-4-[3-(2-methyl-1,3,4-oxadiazol-5-yl)phenyl]-pyridin-3,5-dicarbonsäure-isopropyl-2-methoxyethyl-ester
(-)-1,4-Dihydro-2,6-dimethyl-4-[3-(2-methyl-1,3,4-oxadiazol-5-yl)phenyl]-pyridin-3,5-dicarbonsäure-isopropyl-2-methoxyethyl-ester
(-)-1,4-Dihydro-2,6-dimethyl-4-[3-(1,3,4-oxadiazol-2-yl)phenyl]-pyridin-3,5-dicarbonsäure-isopropyl-2-methoxyethyl-ester
(+)-1,4-Dihydro-2,6-methyl-4-[3-(1,3,4-oxadiazol-2-yl)phenyl]-pyridin-3,5-dicarbonsäure-isopropyl-2-methoxyethyl-ester
1,4-Dihydro-2,6-dimethyl-4-[3-(1,3,4-oxadiazol-2-yl)phenyl]-pyridin-3,5-dicarbonsäure-isopropyl-2-propoxyethyl-ester.

Ganz besonders bevorzugt sind ebenso folgende Verbindungen der allgemeinen Formel (I), in welcher R⁴ für über das Stickstoffatom gebundenes Pyrrol oder 2,5-Dimethylpyrrol steht,
1,4-Dihydro-2,6-dimethyl-4-[3-(pyrrol-1-yl)phenyl]-pyridin-3,5-dicarbonsäure-isopropyl-2-methoxyethyl-ester
1,4-Dihydro-2,6-dimethyl-4-[3-(2,5-dimethyl-pyrrol-1-yl)phenyl]-pyridin-3,5-dicarbonsäure-isopropyl-2-methoxyethyl-ester
4-[2-Chlor-3-(pyrrol-1-yl)phenyl]-1,4-dihydro-2,6-dimethyl-pyridin-3,5-dicarbonsäure-isopropyl-2-methoxyethyl-ester
(+)-4-[2-Chlor-3-(pyrrol-1-yl)phenyl]-1,4-dihydro-2,6-dimethyl-pyridin-3,5-dicarbonsäure-isopropyl-2-methoxyethyl-ester
(-)-4-[2-Chlor-3-(pyrrol-1-yl)phenyl]-1,4-dihydro-2,6-dimethyl-pyridin-3,5-dicarbonsäure-isopropyl-2-methoxyethyl-ester
4-[2-Chlor-5-(pyrrol-1-yl)phenyl]-1,4-dihydro-2,6-dimethyl-pyridin-3,5-dicarbonsäure-isopropyl-2-methoxyethyl-ester
4-[2-Chlor-3-(2,5-dimethylpyrrol-1-yl)phenyl]-1,4-dihydro-2,6-dimethyl-pyridin-3,5-dicarbonsäure-isopropyl-2-methoxyethylester
4-[2-Chlor-5-(2,5-dimethylpyrrol-1-yl)phenyl]-1,4-dihydro-2,6-dimethyl-pyridin-3,5-dicarbonsäure-isopropyl-2-methoxyethyl-ester
4-[2-Chlor-5-(2,5-dimethylpyrrol-1-yl)phenyl]-1,4-dihydro-2,6-dimethyl-pyridin-3,5-dicarbonsäure-isopropyl-cyclopenyl-ester
1,4-Dihydro-2,6-dimethyl-4-[3-(pyrrol-1-yl)phenyl]-pyridin-3,5-dicarbonsäure-cyclopenty-2-methoxyethyl-ester.

Außerdem wurden Verfahren zur Herstellung der erfindungsgemaßen Verbindungen der allgemeinen Formel (I) gefunden, dadurch gekennzeichnet, daß man
[A] Aldehyde der allgemeinen Formel (II) 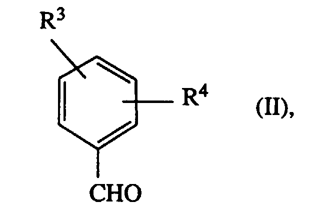 in welcher
   - R³ und R⁴: die oben angegebene Bedeutung haben,
   zunächst mit Acetessigsäureestern der allgemeinen Formel (III)

   H₃C-CO-CH₂-CO₂R² (III),

   in welcher
   - R²: die oben angegebene Bedeutung hat,
   gegebenenfalls unter Isolierung der Benzylidenverbindung umsetzt,
   und anschließend mit 2-Aminocrotonsäureestern der allgmeinen Formel (IV) 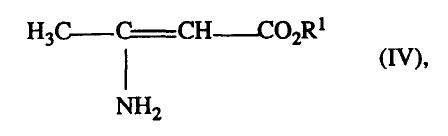 in welcher
   - R¹: die oben angegebene Bedeutung hat
   in inerten Lösemitteln in Anwesenheit einer Base und gegebenenfalls Säuren umsetzt,
   oder
[B] im Fall, daß der Substituent R⁴ für ein 1,3,4-Oxadiazolylrest steht,
   Verbindungen der allgemeinen Formel (V) 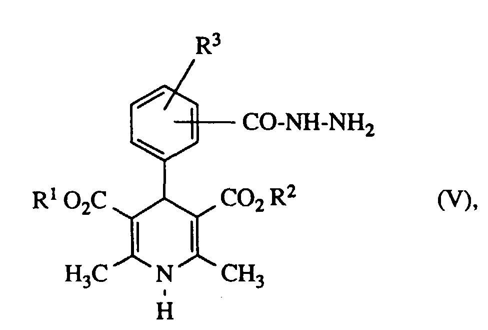 in welcher
   - R¹, R²und R³: die oben angegebene Bedeutung haben,
   mit 1,1,1-Trialkoxyalkanen, und im Fall eines aminosubstituierten Oxadiazolylrestes mit Bromcyan oder Chlorcyan in Gegenwart von Basen umsetzt,
   oder
[C] im Fall, daß R⁴ für ein 1,2,4-Oxadiazolylrest steht,
   Verbindungen der allgemeinen Formel (VI) 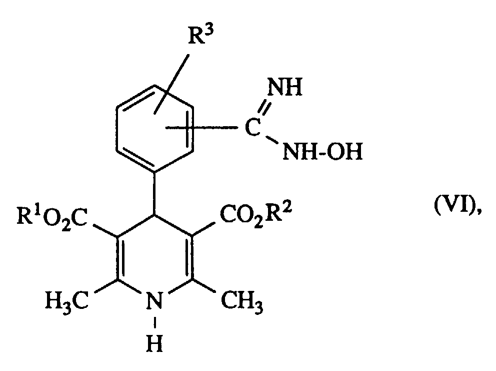 in welcher
   - R¹, R² und R³: die oben angegebene Bedeutung haben,
   mit 1,1,1-Trialkoxyalkanen, Carbonsäurechloriden oder Carbonsäureanhydriden, in inerten Lösemitteln, gegebenenfalls in Anwesenheit von Basen, umsetzt,
   oder
[D] im Fall, daß R⁴ für den Tetrazolylrest steht,
   Verbindungen der allgemeinen Formel (VII) 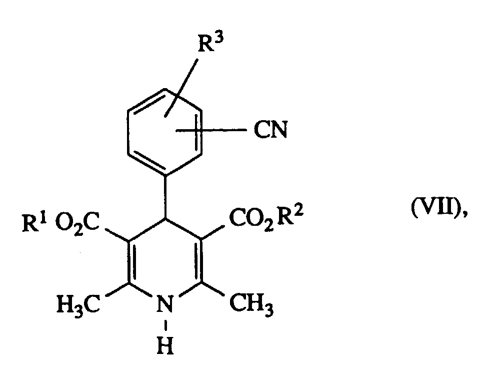 in welcher
   - R¹, R² und R³: die oben angegebene Bedeutung haben,
   in inerten Lösemitteln, mit Natriumazid in Anwesenheit von Ammoniumchlorid umsetzt,
[E] im Fall, daß R⁴ einen über das Stickstoffatom gebundenen Pyrrolrest bedeutet
   Verbindungen der allgemeinen Formel (VIII) 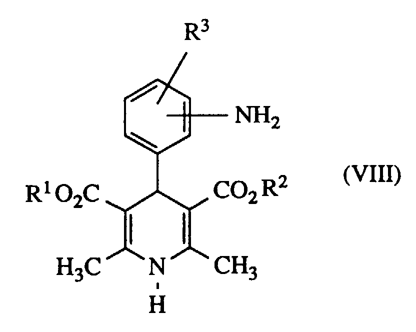 in welcher
   - R¹, R² und R³: die oben angegebene Bedeutung haben,
   in inerten Lösemitteln mit 1,4-Dicarbonylverbindungen der allgemeinen Formel (IX) oder Di(C₁-C₄)alkoxyfuranen der allgemeinen Formel (X) 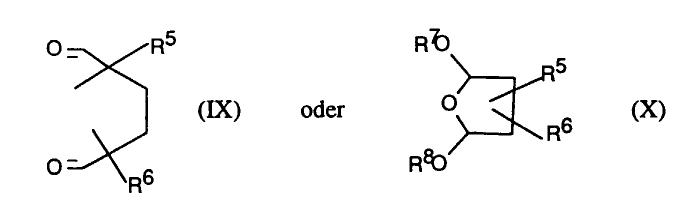 in welcher
   - R⁵ und R⁶: die oben angegebene Bedeutung haben
   und
   - R⁷ und R⁸: gleich oder verschieden sind und C₁-C₄-Alkyl bedeuten,
   in Gegenwart von Säuren umsetzt,
   oder
[F] Verbindungen der allgemeinen Formel (XI) 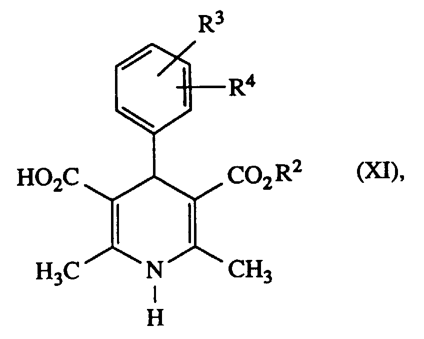 in welcher
   - R², R³ und R⁴: die oben angegebene Bedeutung haben,
   gegebenenfalls über ein reaktives Säurederivat, in Anwesenheit einer Base, mit den entsprechenden Alkoholen umsetzt, wobei durch Einsatz der enantiomerenreinen Derivate der Verbindungen der allgemeinen Formel (XI) die entsprechenden Enantiomeren der allgemeinen Formel (I) erhalten werden.

Die erfindungsgemäßen Verfahren können durch folgendes Formelschema beispielhaft erläutert werden:

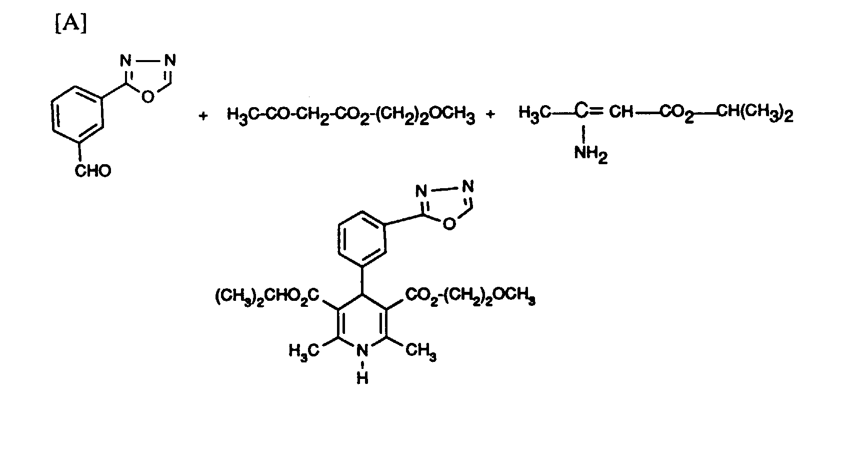

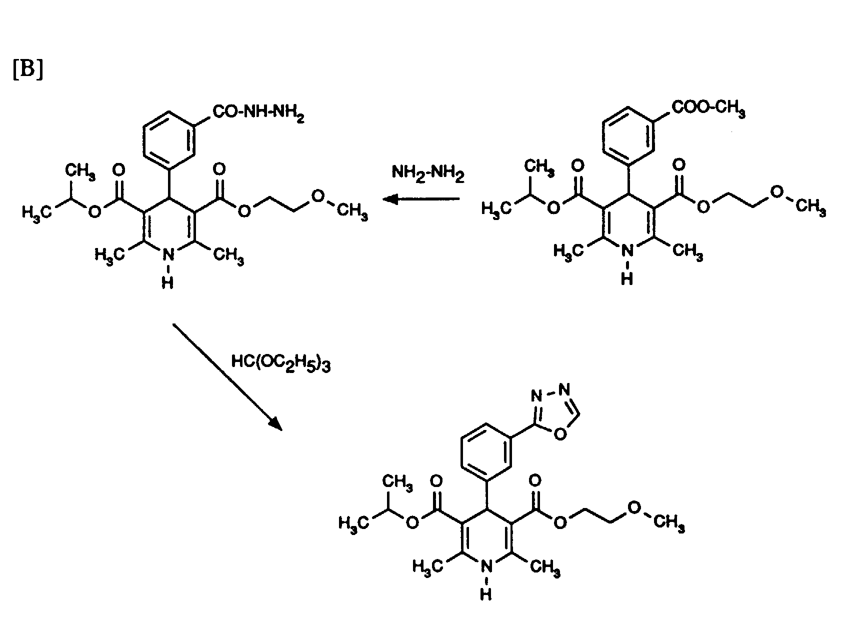

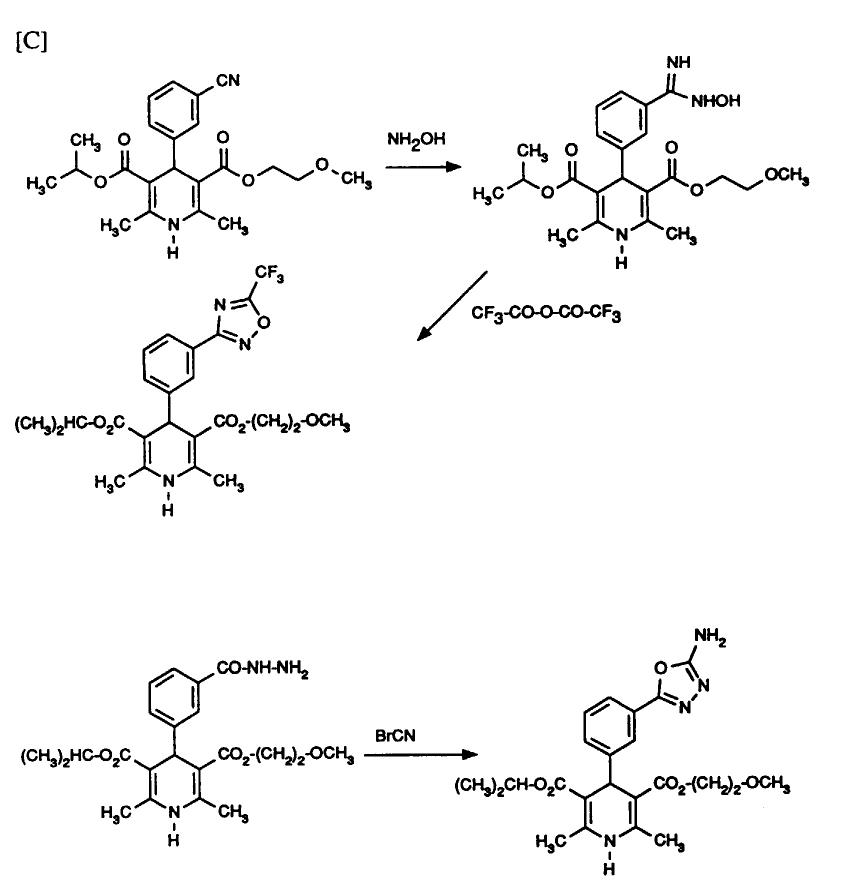

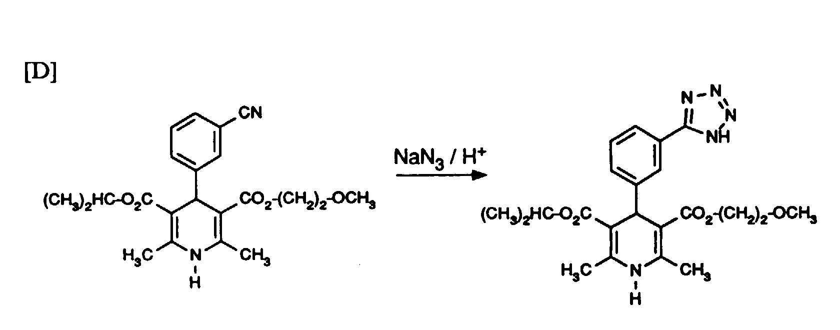

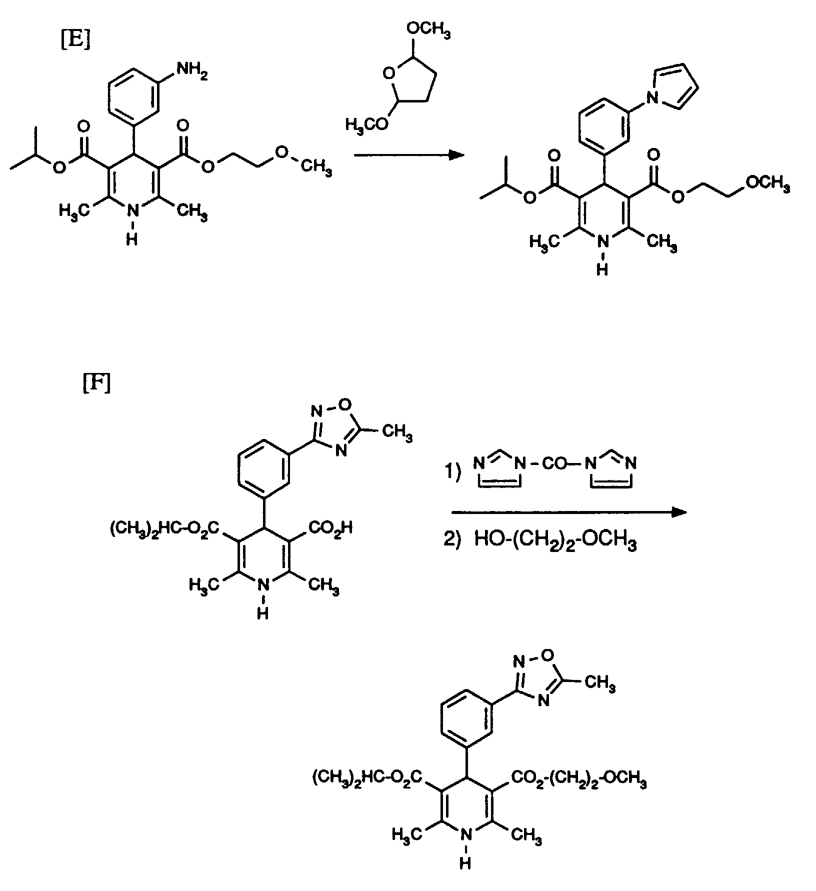

Als Lösemittel für die Verfahren [A] bis [E] eignen sich hierbei alle inerten organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, Tetrahydrofuran, Glykoldimethylether, oder Diethylenglykoldimethylether, Acetonitril, oder Amide wie Hexamethylphosphorsäuretriamid oder Dimethylformamid, oder Essigsäure oder halogenierte Kohlenwasserstoffe wie Methylenchlorid, Tetrachlorkohlenstoff oder Kohlenwasserstoffe wie Benzol oder Toluol. Ebenso ist es möglich, Gemische der genannten Lösemittel zu verwenden. Bevorzugt sind Isopropanol, Ethanol, Tetrahydrofuran, Methanol, Dioxan und Dimethylformamid.

Als Lösemittel für das Verfahren [F] eignen sich die oben aufgeführten Lösemittel mit Ausnahme der Alkohole.

Als Basen eignen sich im allgemeine Alkalihydride oder -alkoholate, wie beispielsweise Natriumhydrid oder Kalium-tert.butylat, oder cyclische Amine, wie beispielsweise Piperidin, Dimethylaminopyridin oder C₁-C₄-Alkylamine, wie beispielsweise Triethylamin. Bevorzugt sind in Abhängigkeit der jeweiligen Reaktionsschritte Piperidin, Dimethylaminopyridin, Pyridin, Natriumhydrid und Kalium-tert.butylat.

Im Fall des Verfahrens [A] eignen sich als Säuren organische C₁-C₃-Carbonsäuren, wie beispielsweise Essigsäure oder Propionsäure. Bevorzugt ist Essigsäure.

Im Falle des Verfahrens [E] eignen sich als Säuren organische C₁-C₃-Carbonsäuren wie beispielsweise Essigsäure oder Propionsäure oder organische C₁-C₇-sulfonsäuren wie Benzolsulfonsäure oder Toluolsulfonsäure. Die organischen Carbonsäuren können im Überschuß als Lösemittel wie auch katalytisch, d.h. mit 0,001 - 1 Molequivalenten bezogen auf Verbindung V eingesetzt werden; organische Sulfonsäuren werden mit 0,001 bis 1 eq eingesetzt. Bevorzugt werden Essigsäure und Toluolsulfonsäure.

Bei der Durchführung der erfindungsgemäßen Verfahren ist das Verhältnis der an der Reaktion beteiligten Stoffe beliebig. Im allgemeinen arbeitet man jedoch bei molaren Mengen der Reaktanden.

Die Reaktionstemperaturen können in einem größeren Bereich variiert werden. Im allgemeinen arbeitet man zwischen +10°C und +150°C, vorzugsweise zwischen +20°C und +100°C, insbesondere bei der Siedetemperatur des jeweiligen Lösemittels.

Die Umsetzungen können bei Normaldruck, aber auch bei erhöhtem oder erniedrigtem Druck (z.B. 0,5 bis 3 bar) durchgeführt werden. Im allgemeinen arbeitet man bei Normaldruck.

Einige Reaktionsschritte werden zweckmäßigerweise unter Schutzgasatmosphäre umgesetzt.

Zur Aktivierung der Carbonsäure eignen sich die üblichen Reagenzien wie anorganische Halogenide, beispielsweise Thionylchlorid, Phosphortrichlorid oder Phosphorpentachlorid, oder Carbonyldiimidazol, Carbodiimide wie Cyclohexylcarbodiimid oder 1-Cyclohexyl-3-[2-(N-methylmorpholino)ethyl]-carbodiimid-p-toluolsulfonat oder N-Hydroxyphthalimid oder N-Hydroxy-benztriazol.

Enantiomerenreine Formen erhält man z.B., indem man Diastereomerengemische der Verbindungen der allgemeinen Formel (I), in welcher R² oder R³ für einen optischaktiven Esterrest steht, nach üblicher Methode trennt, anschließend entweder direkt umestert oder zuerst die optischaktiven Carbonsäuren herstellt und dann durch Veresterung nach Verfahren [E] die enantiomerenreinen Dihydropyridine herstellt.

Geeignet als chirale Esterteste sind alle Ester enantiomerenreiner Alkohole wie beispielsweise 1-Phenylethanol, Milchsäure, Milchsäureester, Mandelsäure, Mandelsäureester, 2-Aminoalkohole, Zuckerderivate, Hydroxyaminosäurederivate und viele andere enantiomerenreine Alkohole mehr.

Die Trennung der Diastereomeren erfolgt im allgemeinen entweder durch fraktionierte Kristallisation, durch Säulenchromatographie oder durch Craig-Verteilung. Welches das optimale Verfahren ist, muß von Fall zu Fall entschieden werden, manchmal ist es auch zweckmäßig, Kombinationen der einzelnen Verfahren zu benutzen. Besonders geeignet ist die Trennung durch Kristallisation oder Craig-Verteilung bzw. eine Kombination beider Verfahren.

Die enantiomerenreinen Verbindungen sind auch zugänglich durch Chromatographie der racemischen Ester auf chiralen Phasen.

Die Verbindungen der allgemeinen Formel (II) sind an sich bekannt oder können nach bekannten Methoden hergestellt werden.

Die Acetessigsäurederivate der allgemeinen Formel (III) sind bekannt oder können nach bekannten Methoden hergestellt werden.

Die Aminocrotonsäurederivate der Formel (IV) sind bekannt oder können nach bekannten Methoden hergestellt werden.

Die Verbindungen der allgemeinen Formel (V) sind als konkrete Stoffvertreter neu und können beispielsweise hergestellt werden, indem man Verbindungen der allgemeinen Formel (XII) 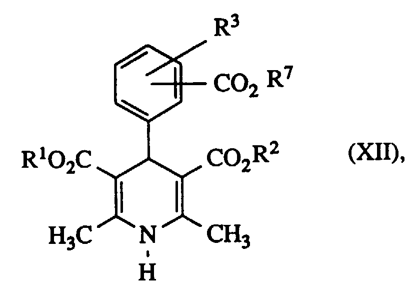 in welcher
- R¹, R² und R³: die oben angegebene Bedeutung haben
und
- R⁷: für C₁-C₄-Alkyl steht,
mit Hydrazinhydrat, in einem der oben angegebenen Lösemitteln, vorzugsweise Methanol und in Anwesenheit von KCN, in einem Temperaturbereich von 30 bis 100°C, bevorzugt bei Rückflußtemperatur und Normaldruck umsetzt.

Die Verbindungen der allgemeinen Formel (VI) sind als konkrete Stoffvertreter neu und können beispielsweise hergestellt werden, indem man Verbindungen der allgemeinen Formel (VII) mit Hydroxylamin oder seinen Salzen, gegebenenfalls in Gegenwart von Basen wie Natriumcarbonat, Kaliumcarbonat oder Triethylamin, in den oben angegebenen Lösemitteln, vorzugsweise in Wasser, Alkoholen oder Gemischen davon, in einem Temperaturbereich von 30°C bis zur Rückflußtemperatur, bevorzugt bei Rückflußtemperatur bei 100°C, und bei Normaldruck umsetzt.

Die Verbindungen der allgemeinen Formel (XII) sind als konkrete Stoffvertreter neu und können dann beispielsweise zunächst durch Umsetzung der entsprechenden 3-Formylbenzoesäureestern mit Acetessigsäureestern in inerten Lösemitteln, vorzugsweise Alkoholen in Anwesenheit von Basen und Säuren, vorzugsweise Piperidin und Eisessig, gegebenenfalls unter Isolierung der entstehenden Ylidenverbindungen und anschließender Reaktion mit dem entsprechenden 2-Aminocrotonsäureester, in einem Temperaturbereich von +30°C bis +100°C, hergestellt werden.

Die Verbindungen der allgemeinen Formeln (VII) sind ebenfalls teilweise bekannt und können nach üblichen Methoden hergestellt werden.

Die Verbindungen der allgemeinen Formel (VIII) sind teilweise neu und können hergestellt werden, indem man Verbindungen der allgemeinen Formel (II) zunächst mit Acetessigestern der allgemeinen Formel (III) und anschließend mit Verbindungen der Formel (IVa) 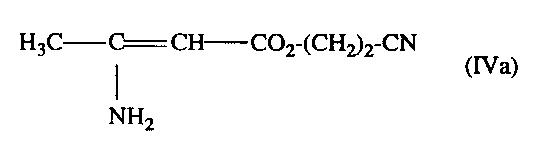 wie unter Verfahren [A] beschrieben versetzt, und den entstandenen Dihydropyridincyanoethylester mit Basen, bevorzugt Natriumhydroxid oder Kaliumtert.butylat behandelt.

Die Verbindungen der allgemeinen Formel (VIII) sind teilweise neu und können beispielsweise hergestellt werden, indem man Verbindungen der allgemeinen Formel (XIII) 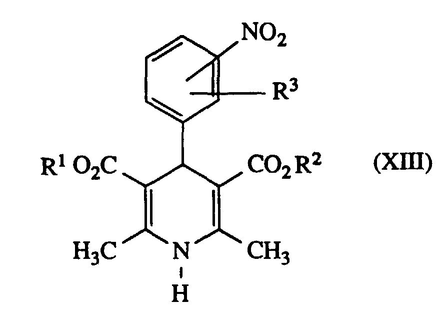 in welcher
- R¹, R² und R³: die oben angegebene Bedeutung haben,
in Gegenwart eines Katalysators, vorzugsweise Raney-Nickel, in einem Alkohol, vorzugsweise Methanol, bei Normaldruck und Raumtemperatur hydriert.

Die Verbindungen der allgemeinen Formel (XIII) sind teilweise bekannt und können dann beispielsweise zunächst durch Umsetzung der entsprechenden 3-Formylbenzoesäureester mit Acetessigsäure-estern in inerten Lösemitteln, vorzugsweise Alkoholen in Anwesenheit von Basen und Säuren, vorzugsweise Piperidin und Eisessig, gegebenenfalls unter Isolierung der entstehenden Ylidenverbindungen und anschließender Reaktion mit dem entsprechenden 2-Aminocrotonsäureester, in einem Temperaturbereich von +30°C bis +100°C, hergestellt werden.

Die Verbindungen der allgemeinen Formeln (IX) und (X) sind bekannt oder können nach üblichen Methoden hergestellt werden.

Die vorstehende Herstellungsverfahren sind lediglich zur Verdeutlichung angegeben. Die Herstellung der Verbindungen der allgemeinen Formel (I) ist nicht auf diese Verfahren beschränkt, sondern jede Modifikation dieser Verfahren ist in gleicher Weise für die Herstellung der erfindungsgemäßen Verbindungen anwendbar.

Die erfindungsgemäßen Verbindungen zeigen ein nicht vorhersehbares, wertvolles pharmakologisches Wirkspektrum.

Die erfindungsgemäßen Verbindungen sind Calciumkanalliganden mit Selektivität für L-Typ-Calciumkanäle des zentralen Nervensystems. Diese Selektivität läßt sich z.B. durch Vergleich der Bindungsaffinitäten zu DHP-Bindungstellen an Rattenhirn und Rattenherz zeigen.

Die Verbindungen haben einen positiven Einfluß auf Lern- und Gedächtnisleistungen, wie ihre leistungsverbessernde Wirkung auf Ratten in typischen Lern- und Gedächtnismodellen (z.B. Wasser-Labyrinth, Morris-Labyrinth, Passives Vermeiden, Reminiszenztests in automatisierten Skinner-Boxen) demonstriert. Sie besitzen ein antidepressives Potential, wie ihre Wirksamkeit im Rattenschwimmtest nach Porsolt belegt.

### Bindungsassays:

Die Bindungsaffinitäten zu PN 200-110-Bindungsstellen in Rattenhirnen bzw. Rattenherzen werden nach Rampe D.R., Mutledge A., Janis R.A., Triggle D.J.: Can. Journ. Physiol. Pharmacol. 65, (1987) 1452 bestimmt.

### Wasserlabyrinth:

Alte Wistar-Ratten (24 Monate) werden an die Startposition in einem mit kaltem (14-15°) Wasser gefüllten, durch senkrechte Barrieren unterteilten Plastiktank (120x50x40 cm) gesetzt. Um zu einer Leiter zu gelangen, die den Tieren das Entkommen aus dem Wasser ermöglicht, müssen sie um diese Barrieren herumschwimmen. Die Zeit, die zum Auffinden des Ausstiegs benötigt wird und die Zahl der Fehler auf dem Weg dorthin werden aufgezeichet. Dabei wird ein Fehler definiert als Schwimmen in eine Sackgasse oder Schwimmen über die Grenzlinie imaginärer Quadrate, in die der Tank unterteilt ist, in die Richtung fort vom Ausstieg.
Die Ratten bleiben bis zum Finden des Ausgangs, längstens aber 300 sec. im Labyrinth. Dann werden sie aufgenommen, getrocknet und unter einem Rotlicht gewärmt. Danach kehren sie in ihre Heimatkäfige zurück.
In einem typischen Experiment werden durch einen Vortest zwei äquivalente Tiergruppen (Placebo, Testsubstanz je n = 15) ermittelt. Anschließend durchlaufen die Tiere 6 Testsitzungen, zwei je Tag. Testsubstanzen bzw. Placebo werden 30 min. vor den Versuchen per os verabreicht. Maß für die lern- und gedächtnisverbessernde Wirkung der Testsubstanzen im Vergleich zu Placebo sind Verkürzung der Zeit bis zum Erreichen des Ausstiegs, Verringerung der Zahl der Fehler und Vergrößerung der Zahl der Tiere, die den Ausstieg überhaupt finden.

### Rattenschwimmtest nach Porsolt

Während eines Vortests werden junge Ratten in einem Glaszylinder (40 xm hoch, 20 cm Durchmesser) gesetzt, der 17 cm hoch mit Wasser von 25°C gefüllt ist Nach 20 min im Wasser werden die Tiere herausgenommen und unter einer Lampe 30 min gewärmt. In diesem Vortest versuchen alle Ratten, aus dem Zylinder zu entkommen, bis sie nach etwa 15 min immobil verharren ("behavioral despair", Aufgabeverhalten). 24 h später beginnt die Testsitzung in der die Ratten wie am Vortag in den Glaszylinder gesetzt werden, jedoch diesmal nur 5 min. Die Zeitspannen, die die Ratten während dieser 5 min immobil verharren, werden aufgezeichnet Dabei wird eine Ratte als immobil angesehen, die aufrecht im Wasser treibend nur noch minimale Bewegungen ausführt, um den Kopf über Wasser zu halten. Placebo bzw. Testsubstanzen (0,25, 0,5, 1, 5, 10 mg/kg; n = 6 je Gruppe) werden dreimal per os verabreicht: 23, 5 und 1 h vor der Testsitzung (1, 19, 23 h nach dem Vortest). Die antidepressive Wirkung der Testsubstanzen zeigt sich in der Reduktion der Immobilitätsdauer im Vergleich zu den Placebowerten.

Aufgrund ihrer pharmakologischen Eigenschaften können sie für die Herstellung von Arzneimitteln zur Behandlung von zentral degenerativen Erkrankungen eingesetzt werden, wie z.B. Auftreten bei Demenzen (Multiinfarktdemenz, MID, primär degenerativer Demenz PDD, prä- und seniler Alzheimerscher Krankheit, HIV-Demenz und andere Demenzformen), Parkinsonscher Krankheit oder amyotrophischer Lateralsklerose.

Weiterhin eignen sich die Wirkstoffe zur Behandlung von Hirnleistungsstörungen im Alter, des himorganischen Psychosyndroms (HOPS, Organic brain syndrom, OBS) und von altersbedingten Gedächtnisstörungen (age associated memory impairment, AAMI).

Sie sind wertvoll zur Prophylaxe und zur Bekämpfung der Folgen cerebraler Durchblutungsstörungen wie cerebraler Ischämien, Schlaganfallen, von Subarachnoidalblutungen und zur Behandlung von Hirntraumen.

Sie sind geeignet zur Behandlung von Depressionen und der Manie. Weitere Anwendungsgebiete sind die Behandlung von Migräne, von Neuropathien, von Suchterkrankungen und Entzugserscheinungen.

Zur vorliegenden Erfindung gehören auch pharmazeutische Zubereitungen, die neben inerten, nicht-toxischen, pharmazeutisch geeigneten Hilfs- und Trägerstoffen eine oder mehrere Verbindungen der allgemeinen Formel (I) enthalten, oder die aus einem oder mehreren Wirkstoffen der Formel (I) bestehen, sowie Verfahren zur Herstellung dieser Zubereitungen.

Die Wirkstoffe der Formel (I) sollen in diesen Zubereitungen in einer Konzentration von 0,1 bis 99,5 Gew.-%, bevorzugt von 0,5 bis 95 Gew.-% der Gesamtmischung vorhanden sein.

Neben den Wirkstoffen der Formel (I) können die pharmazeutischen Zubereitungen auch andere pharmazeutische Wirkstoffe enthalten.

Die oben aufgeführten pharmazeutischen Zubereitungen können in üblicher Weise nach bekannten Methoden hergestellt werden, beispielsweise mit dem oder den Hilfs- oder Trägerstoffen.

Im allgemeinen hat es sich als vorteilhaft erwiesen, den oder die Wirkstoffe der Formel (I) in Gesamtmengen von etwa 0,01 bis etwa 100 mg/kg, bevorzugt in Gesamtmengen von etwa 1 mg/kg bis 50 mg/kg Körpergewicht je 24 Stunden, gegebenenfalls in Form mehrerer Einzelgaben, zur Erzielung des gewünschten Ergebnisses zu verabreichen.

Es kann aber gegebenenfalls vorteilhaft sein, von den genannten Mengen abzuweichen, und zwar in Abhängigkeit von der Art und vom Körpergewicht des behandelten Objekts, vom individuellen Verhalten gegenüber dem Medikament, der Art und Schwere der Erkrankung, der Art der Zubereitung und Applikation, sowie dem Zeitpunkt bzw. Intervall, zu welchem die Verabreichung erfolgt.

Die jeweils aufgeführten R_{f}-Werte wurden - sofern nicht anders vermerkt - durch Dünnschichtchromatographie auf Kieselgel (Alufolie, Kieselgel 60 F 254, Fa. E. Merck) ermittelt. Die Visualisierung der Substanzflecke geschah durch Betrachten unter UV-Licht und/oder durch Besprühen mit 1%iger Kaliumpermanganat-Lösung.

Die Flash-Chromatographie wurde auf Kieselgel 60, 0,040 - 0,064 mm, Fa. E. Merck, durchgeführt. Elution mit Lösemittelgradienten bedeutet: Beginnend mit der reinen, unpolaren Lösemittelgemischkomponente wird in einem steigendem Ausmaß die polare Laufmittelkomponente zugemischt, bis das gewünschte Produkt eluiert wird (DC-Kontrolle).

### Ausgangsverbindungen

### Beispiel I

1,4-Dihydro-2,6-dimethyl-4-(3-methoxycarbonyl-phenyl)-pyridin-3,5-dicarbonsäure-isopropyl-2-methoxyethyl-ester 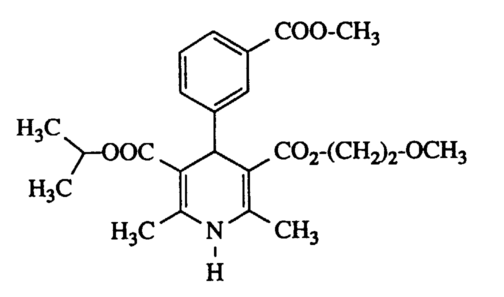 10,0 g (61 mmol) 3-Formylbenzoesäuremethylester, 10,0 g (60 mmol) Acetessigsäure-2-methoxyethylester, 0,2 ml Eisessig und 0,4 ml Piperidin werden in 100 ml 2-Propanol 2,5 h auf 40°C erhitzt. Nach Abkühlen wird eingeengt und das Rohprodukt durch Flashchromatographie (Kieselgel, Toluol/Essigester 20:1 bis 1:1) gereinigt Man erhält 17,6 g (94%) Benzylidenverbindung als gelbes Öl.
17,6 g (57,5 mmol) dieses Zwischenproduktes und 8,2 g (75,5 mmol) 3-Aminocrotonsäureisopropylester in 150 ml 2-Propanol werden 1 h zum Rückfluß erhitzt. Das nach Einengen erhaltene Rohprodukt wird durch Chromatographie (Kieselgel, Toluol/Essigester 1:0 bis 1:1) gereinigt. Man erhält so 19.8 g (80%) Produkt, das für weitere Umsetzung von hinreichender Reinheit ist. Durch Verreiben mit Diethylether läßt sich das Produkt in Form farbloser Kristalle, Schmp. 86-88°C, gewinnen.

In Analogie zur Vorschrift des Beispiels I werden die in Tabelle I aufgeführten Verbindungen erhalten:

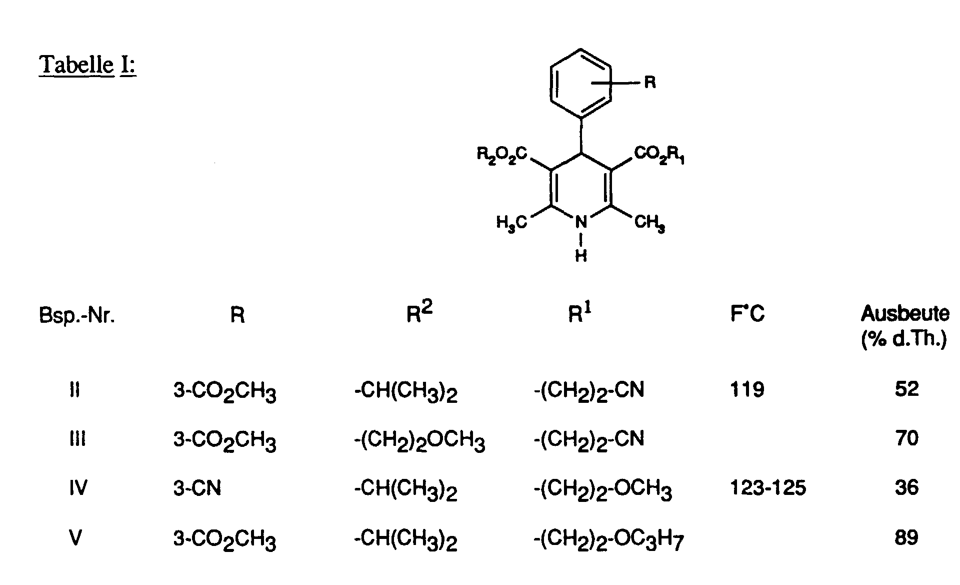

### Beispiel VI

1,4-Dihydro-2,6-dimethyl-4-(3-hydrazinocarbonyl-phenyl)-pyridin-3,5-dicarbonsäure-isopropyl-2-methoxyethyl-ester 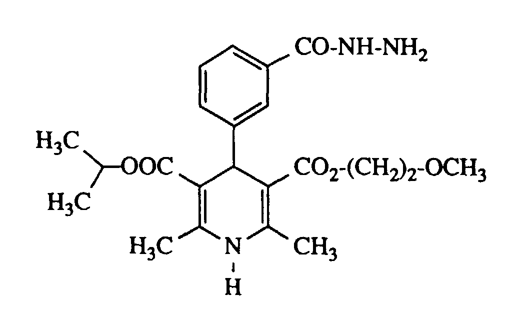 3,0 g (7,0 mmol) der Verbindung aus Beispiel I und 3,0 ml wäßrige Hydrazinhydratlösung (64%ig) werden in 30 ml Methanol gelöst und mit einer Spatelspitze Kaliumcyanid versetzt. Nach 2 h Erhitzen zum Rückfluß werden nochmals 3,0 ml Hydrazinhydratlösung zugesetzt und weitere 60 min zum Rückfluß erhitzt. Das Reaktionsgemisch wird im Vakuuum vom Lösemittel befreit. Digerieren des Rückstandes mit Essigsäureethylester ergibt 2,3 g (77%) farblose Kristalle.
Schmp.: 187-189°C.
Die Substanz wird bei +4°C gelagert.

### Beispiel VII

1,4-Dihydro-2,6-dimethyl-4-(3-methoxycarbonyl-phenyl)-pyridin-3,5-dicarbonsäure-2-methoxy-ethyl-ester 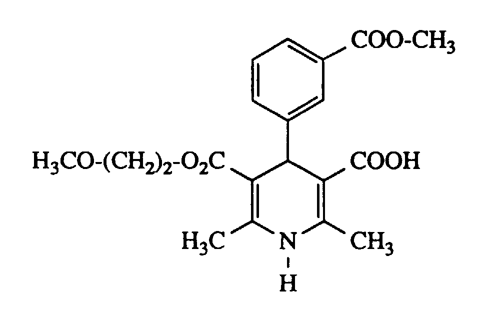 100 g (0,23 mol) der Verbindung aus Beispiel III in 1 l Tetrahydrofuran werden unter Argon bei Raumtemperatur portionsweise mit 30,4 g (0,27 mol) Kalium-tert.butylat versetzt. Nach 2 h bei Raumtemperatur wird auf 50°C erhitzt und die Lösung mit einem leichten Vakuum auf ca. 2/3 eingeengt. Nach Verdünnen mit 2 l Essigsäureethylester wird auf ein Gemisch aus 2 l Wasser und 30 ml konz Salzsäure gegossen. Nach Filtration über Kieselgur wird die wäßrige Phase abgetrennt und die organische Phase gewaschen und getrocknet. Verrühren der organischen Phase mit Tonsil und Einengen liefern ein erstarrendes Öl, das durch Verreiben mit Diethylether in farblose Kristalle, Schmp. 152°C (unter Gasentw.) überführt wird. Ausbeute: 77,4 g (88%)

In Analogie zur Vorschrift des Beispiels VII werden die in Tabelle II aufgeführten Verbindungen hergestellt:

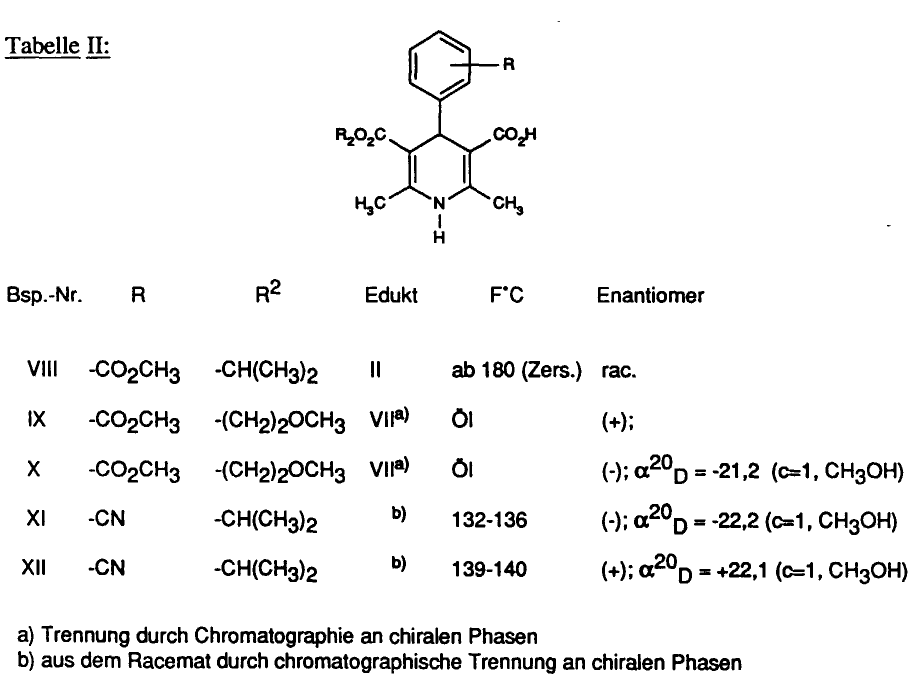

### Beispiel XIII

1,4-Dihydro-2,6-dimethyl-4-(3-methoxycarbonyl-phenyl)-pyridin-3,5-dicarbonsäure-di-(2-methoxyethyl)-ester 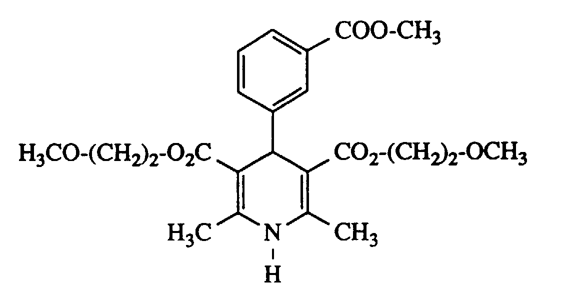 0,40 g (1,0 mmol) der Verbindung aus Beispiel VII in 4 ml abs. Tetrahydrofuran werden mit 0,16 g (1,0 mmol) Carbonyldiimidazol versetzt und über Nacht bei Raumtemperatur gerührt. Das Lösemittel wird am Rotationsverdampfer abgezogen, und der Rückstand mit 4 ml 2-Methoxyethanol aufgenommen. Es wird eine Spatelspitze 4-Dimethylaminopyridin zugesetzt und 24 h auf 80°C erhitzt. Das nach Einengen verbleibende Rohprodukt wird durch Chromatographie gereinigt (Kieselgel, Toluol/Essigester 10:1 bis 3:1). Man erhält 0,35 g (78%) Titelverbindung als Öl.

In Analogie zur Vorschrift des Beispiels XIII werden den in Tabelle III aufgeführten Verbindungen hergestellt:

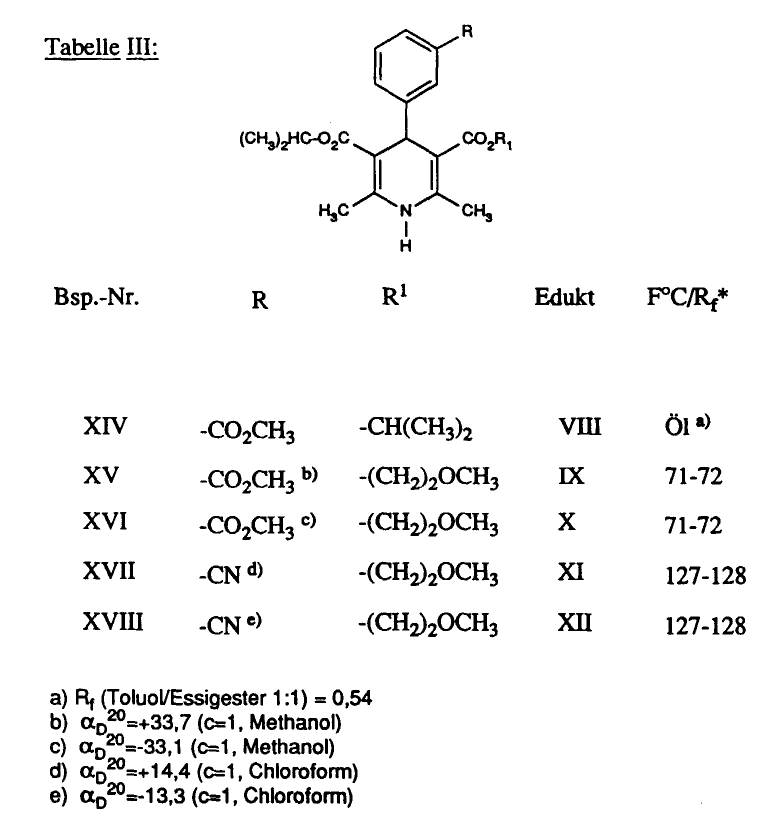

In Analogie zur Vorschrift des Beispiels VI werden die in Tabelle IV aufgeführten Verbindungen hergestellt:

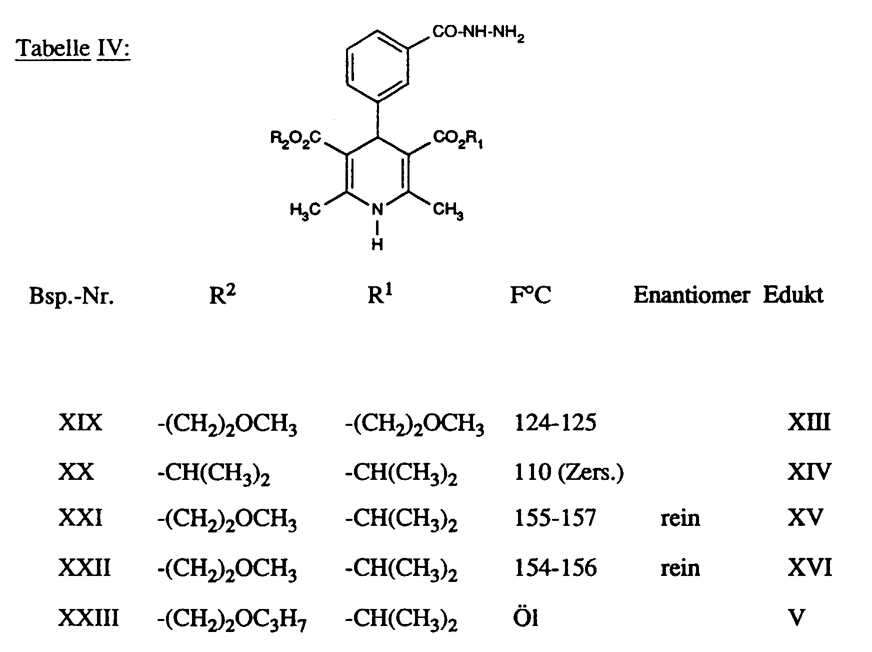

### Beispiel XXIV

1,4-Dihydro-2,6-dimethyl-4-(3-N-hydroxycarbonimidamido-phenyl)-pyridin-3,5-dicarbonsäure-isopropyl-2-methoxyethyl-ester 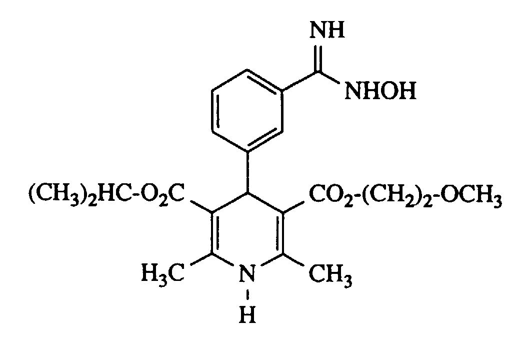 3,98 g (10 mmol) der Verbindung aus Beispiel IV, 1,40 g (20 mmol) Hydroxylamin-hydrochlorid und 1,40 g (10 mmol) Kaliumcarbonat werden in 40 ml Ethanol/Wassergemisch 4:1 suspendiert und über Nacht zum Rückfluß erhitzt. Das Reaktionsgemisch wird eingeengt, der Rückstand wird mit Essigester ausgelaugt. Nach Einengen erhält man aus den Extrakten 4,2 g Titelverbindung (97%) als amorphen Feststoff, der roh weiter umgesetzt wird.

Analog zu Beispiel XXIV werden die in Tabelle V aufgeführten Verbindungen erhalten:

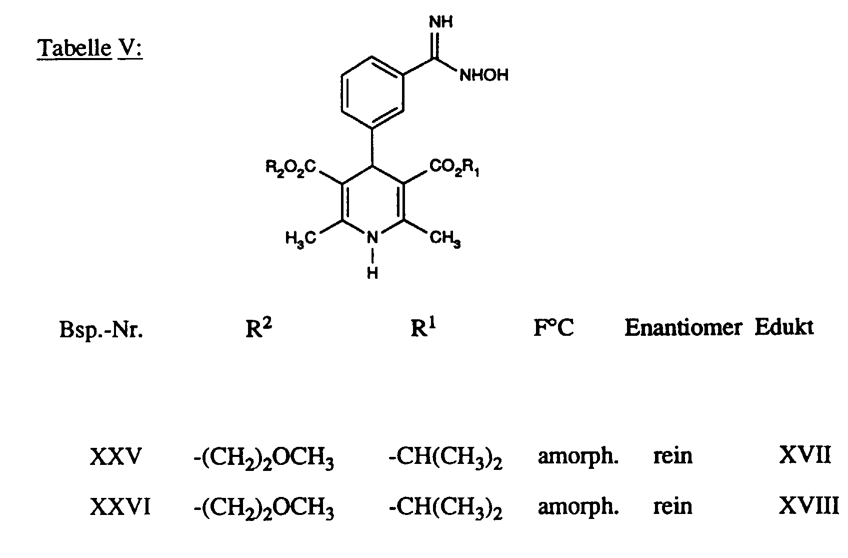

### Beispiel XXVII

4-(3-Amino-2-chloro-phenyl)-1,4-dihydro-2,6-dimethyl-pyridin-3,5-dicarbonsäure-isopropyl-2-methoxyethyl-ester 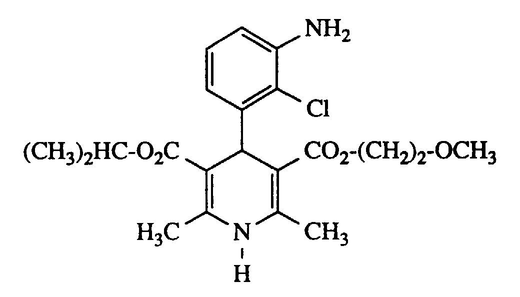 27,5 g (61 mmol, Reinheit 96,5%) 4-(2-Chloro-3-nitrophenyl)-1,4-dihydro-2,6-dimethyl-pyridin-3,5-dicarbonsäure-isopropyl-2-methoxyethyl-ester, erhalten durch Umsetzen equimolarer Mengen 2-Chlor-3-nitrobenzaldehyd, β-Aminocrotonsäure-isopropylester und Acetessigsäuremethoxyethylester in 2-Propanol bei Rückflußtemperatur, werden in 500 ml Methanol suspendiert und in Gegenwart von Raney-Nickel (ca. 1,5 g, wasserfeucht) bei Normaldruck und -temperatur bis zum Ende der Wasserstoffaufnahme hydriert (1h). Der Katalysator wird abfiltriert und der nach Einengen erhaltene Rückstand durch Flashchromatographie (Toluol/Essigester 1:0 -> 5:1) gereinigt. Kristallisation aus Toluol ergibt 24,0 g (93% d.Th.) der Titelverbindung vom Schmp. 118-122°C.

### Beispiel XXVIII

4-(5-Amino-2-chlorophenyl)-1,4-dihydro-2,6-dimethyl-pyridin-3,5-dicarbonsäure-isopropyl-2-methoxyethylester 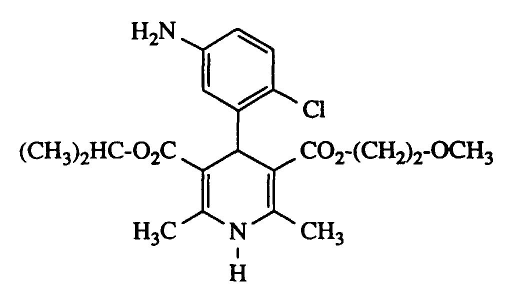 In Analogie zur Vorschrift des Beispiels XXVII wird die Titelverbindung aus 22,8 g (50 mol) 4-(2-Chloro-5-nitrophenyl)-1,4-dihydro-2,6-dimethyl-pyridin-3,5-dicarbonsäure-isopropyl-2-methoxyethylester erhalten.
R_{f} = 0,23 (Toluol/Essigester 1:1)

### Beispiel XXIX

4-(3-Aminophenyl)-1,4-dihydro-2,6-dimethyl-pyridin-3,5-dicarbonsäure-2-cyanoethyl-2-methoxyethylester

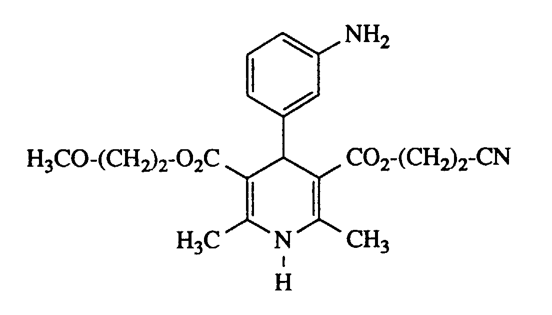

Die Titelverbindung wird in Analogie zu Beispiel XXVII aus 49,0 g (114 mmol) 1,4-Dihydro-2,6-dimethyl-4-(3-nitrophenyl)-pyridin-3,5-dicarbonsäure-2-cyanoethyl-2-methoxyethylester erhalten und als Rohprodukt direkt weiter umgesetzt.

### Beispiel XXX

4-(3-Aminophenyl)-1,4-dihydro-2,6-dimethyl-pyridin-3,5-dicarbonsäure-isopropyl-2-methoxyethylester

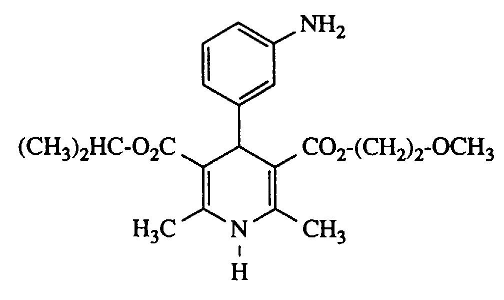

Die Titelverbindung wird durch Umsetzung von 12,6 g (30,1 mmol) 1,4-Dihydro-2,6-dimethyl-4-(3-nitrophenyl)-pyridin-3,5-dicarbonsäure-isopropyl-2-methoxyethyl-ester in Analogie zu Beispiel XXVII erhalten.
FP°C: 138-140°C

### Beispiel XXXI

4-(2-Chlor-3-nitrophenyl)-1,4-dihydro-2,6-dimethyl-pyridin-3,5-dicarbonsäure-isopropylmonoester 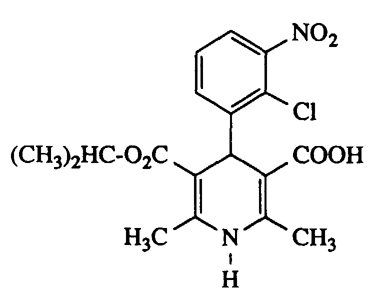 Aus 55,7 g (300 mmol) 2-Chlor-3-nitrobenzaldehyd, 43,2 g (300 mmol) Acetessigsäureisopropylester und 46,2 g (300 mmol) 3-Aminocrotonsäure-2-cyanoethylester werden auf übliche Weise 45,6 g (34%) 4-(2-Chloro-3-nitrophenyl)--1,4-dihydro-2,6-dimethyl-pyridin-3,5-dicarbonsäure-2-cyanoethyl-isopropylester erhalten, die in 500 ml Dimethoxyethan gelöst und mit 5,3 g Natriumhydroxid in 100 ml Wasser versetzt werden. Nach 2 h bei Raumtemperatur wird auf 6 l Eiswasser gegossen, dem 20 ml konzentrierte Salzsäure zugesetzt werden. Der sich abscheidende Niederschlag wird nach 30 Minuten abgesaugt, gewaschen und getrocknet. Man erhält so 39,2 g fast farblose Kristalle.
Schmp.: 159°C

### Beispiel XXXII

4-(3-Amino-2-chlorophenyl)-1,4-dihydro-2,6-dimethyl-pyridin-3,5-dicarbonsäure-isopropyl-2-methoxyethylester (Isomer A) 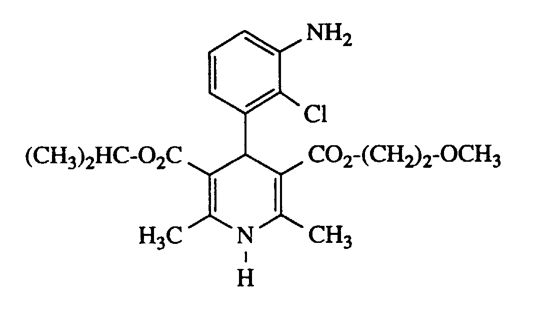 Durch Chromatographie an chiralem Trägermaterial läßt sich aus Beispiel XXXI das reine (+)-Enantiomere gewinnen (α²⁰ _{D}= +21,0; c=1, THF); 5,9 g (15 mmol) hiervon in 60 ml Tetrahydrofuran werden mit 2,4 g (15 mmol) Carbonyldiimidazolid versetzt und 1 h bei 60°C gerührt. Nach Einengen wird mit 40 ml 2-Methoxyethanol versetzt und 18 h bei 80°C gerührt. Das Lösemittel wird entfernt und der Rückstand durch Flashchromatographie (Toluol/Essigester 1:0 bis 3:1) gereinigt, Kristallisation aus Toluol/Cyclohexan liefert 4,3 g (63% d.Th.) 4-(2-Chlor-3-nitrophenyl)-1,4-dihydro-2,6-dimethyl-pyridin-3,5-dicarbonsäure-isopropyl-3-methoxyethylester (α²⁰ _{D}= -10,1; c=0,9, CHCl₃).
Analog zu Beispiel I werden hieraus 4,0 g (quantitativ) der Titelverbindung als Rohprodukt erhalten, das direkt weiter umgesetzt wird.

### Herstellungsbeispiele

### Beispiel 1

1,4-Dihydro-2,6-dimethyl-4-[3-(1,3,4-oxadiazol-2-yl)-phenyl]-pyridin-3,5-dicarbonsäure-isopropyl-2-methoxyethyl-ester 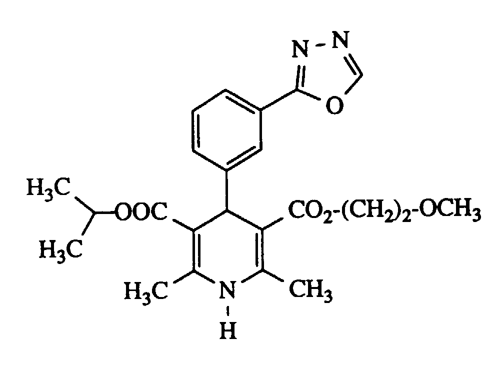 4,4 g (10 mmol) der Verbindung aus Beispiel VI werden mit 20 ml Triethoxymethan versetzt und 30 min zum Rückfluß erhitzt, entstehendes Ethanol wird dabei kontinuierlich abdestilliert. Nach Einengen wird das Rohprodukt durch Chromatographie (Kieselgel, Toluol/Essigester 1:0 bis 1:1) gewonnen. Durch Verreiben mit Diethylether erhält man Kristalle vom Schmp. 134-136°C, die aus Toluol umkristallisiert werden. Dies ergibt 2,0 g (44%) Titelverbindung in Form farbloser Kristalle.
Schmp.: 138-140°C

In Analogie zur Vorschrift des Beispiels 1 werden die in den Tabellen 1 und 2 aufgeführten Verbindungen hergestellt:

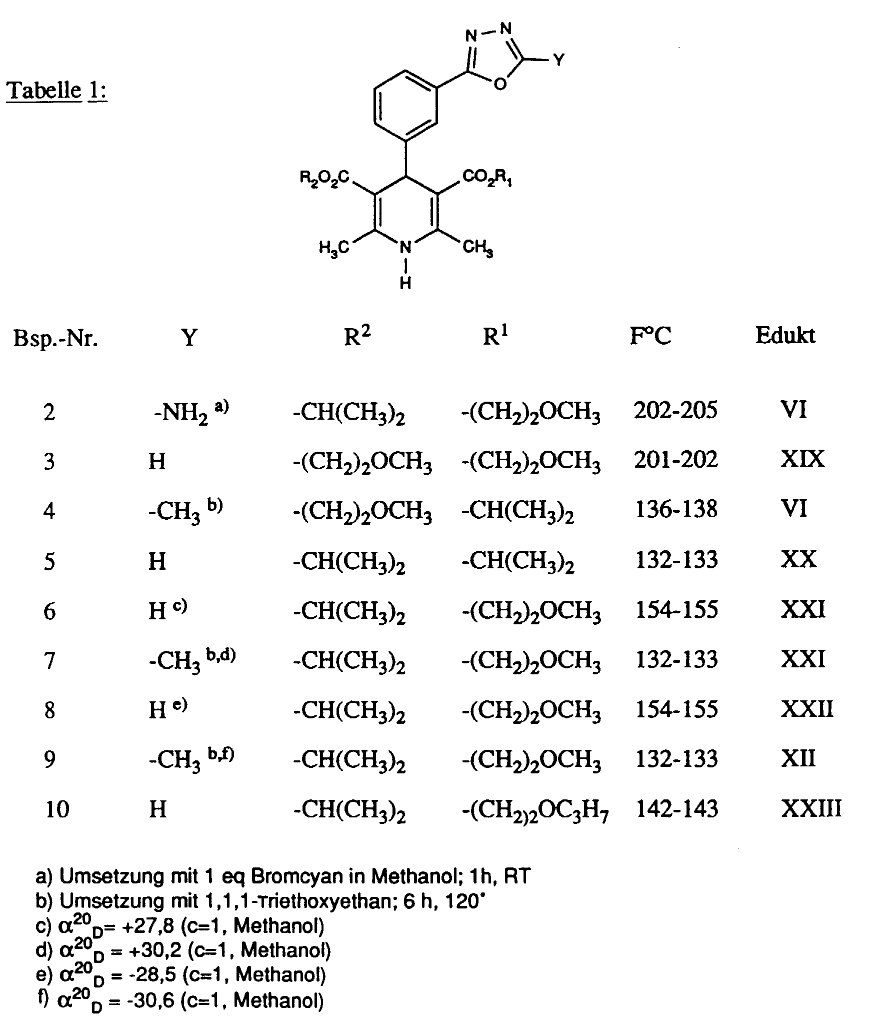

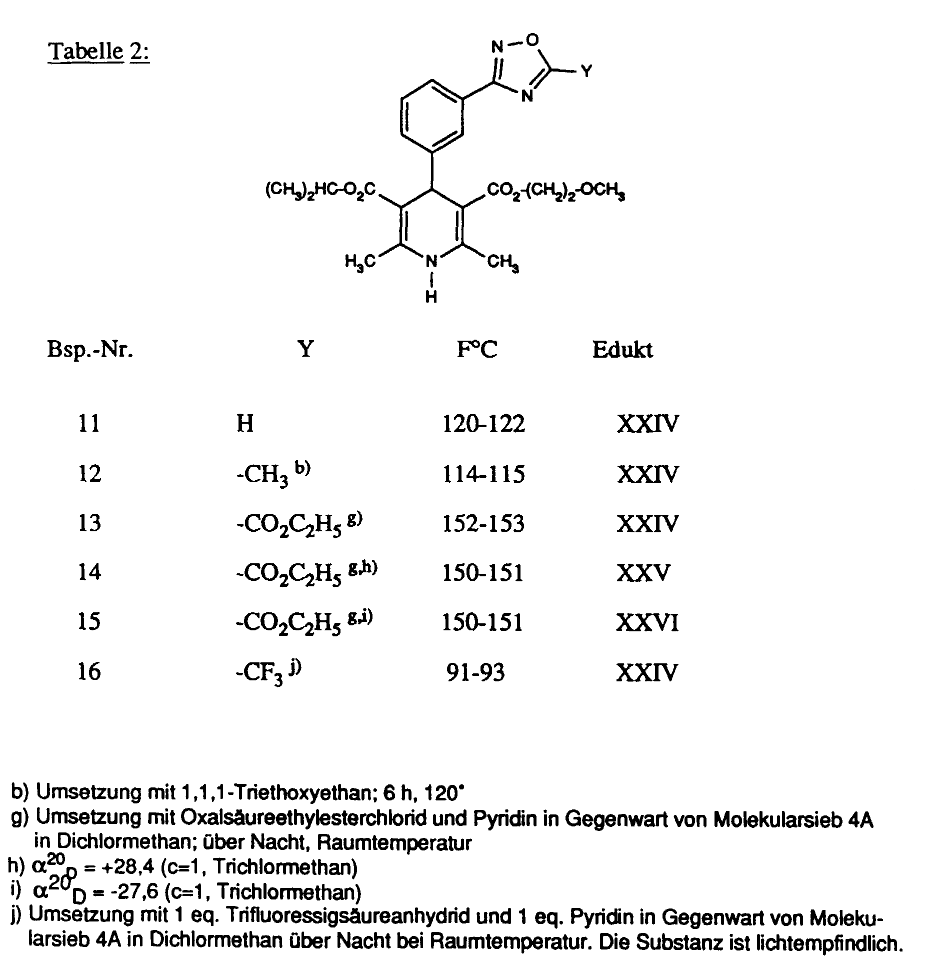

### Beispiel 17

1,4-Dihydro-2,6-dimethyl-4-(3-tetrazol-5-yl-phenyl)-pyridin-3,5-dicarbonsäure-isopropyl-2-methoxyethyl-ester 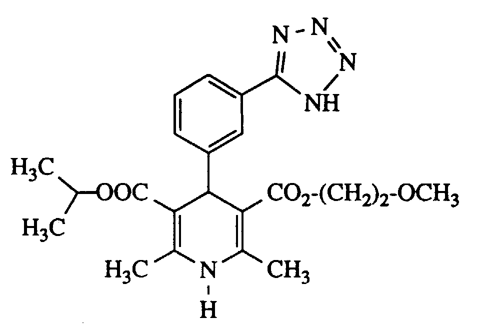 3,98 g (10 mmol) der Verbindung aus Beispiel IV in 40 ml trockenem Dimethylformamid werden mit 0,71 g (11 mmol) Natriumazid und 0,59 g (11 mmol) Ammoniumchlorid versetzt. Nach 16 h bei 100°C wird die erkaltete Mischung auf 120 ml Wasser gegossen. Die Lösung wird mit konzentrierter Salzsäure auf pH 1 gebracht und mehrmals mit Essigsäureethylester extrahiert. Trocknen und Einengen der organischen Phase ergibt das Rohprodukt, das durch zweimalige Chromatographie (Kieselgel, Dichlormethan/2-Propanol 1:0 bis 1:1 bzw. Toluol / Essigester 1:0 bis 0:1) gereinigt wird. Verreiben der eingedampften Eluate liefert 1,80 g (41%) farblose Kristalle, Schmp. >129°C (unter Gasentwicklung).

### Beispiel 18

1,4-Dihydro-2,6-dimethyl-4-[(3-pyrrol-1-yl)phenyl]-3,5-dicarbonsäure-isopropyl-2-methoxyethylester 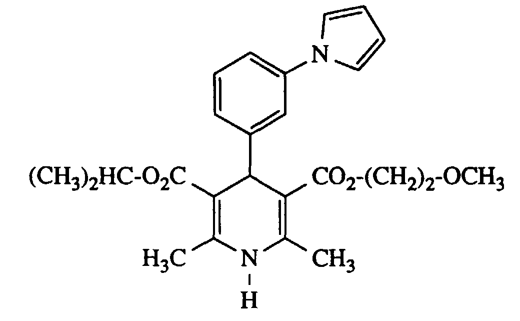 Unter Argon werden 3,2 g (8,2 mmol) der Verbindung aus Beispiel XXX in 11 ml Eisessig gelöst und mit 1,1 g (8,2 mmol) 2,5-Dimethoxytetrahydrofuran versetzt. Es wird zunächst 30 min auf 40°C, anschließend 30 min auf 60°C erhitzt. Es werden 100 ml Chloroform und nochmals die gleiche Menge 2,5-Dimethoxytetrahydrofuran zugegeben. Das Azeotrop Methanol/Chloroform wird abdestilliert; zu dem auf 50 ml eingeengten Reaktionsgemisch werden nochmals 8,2 mmol 2,5-Dimethoxytetrahydrofuran zugegeben. Nach 2 Stunden bei Rückfluß wird eingeengt, und mit 4 ml Eisessig aufgenommen. Nach Zugabe von 1,1 g (8,2 mmol) 2,5-Dimethoxytetrahydrofuran wird 30 min auf 90°C erhitzt. Nach Abkühlen wird mit 100 ml Essigester verdünnt und auf gesättigte Natriumbicarbonatlösung gegossen. Die organische Phase liefert nach Trocknen und Einengen 6,0 g eines Öls, das durch Flaschchromatographie (Toluol/Essigester 3:1) gereinigt wird. Man erhält 1,9 g Öl, das durch Verreiben mit Diisopropylether in 1,1 g farblose Kristalle (30% d.Th.) vom Schmp. 117-119°C überführt wird.

Auf gleiche Weise werden die in Tabelle 1 aufgeführten Verbindungen erhalten:

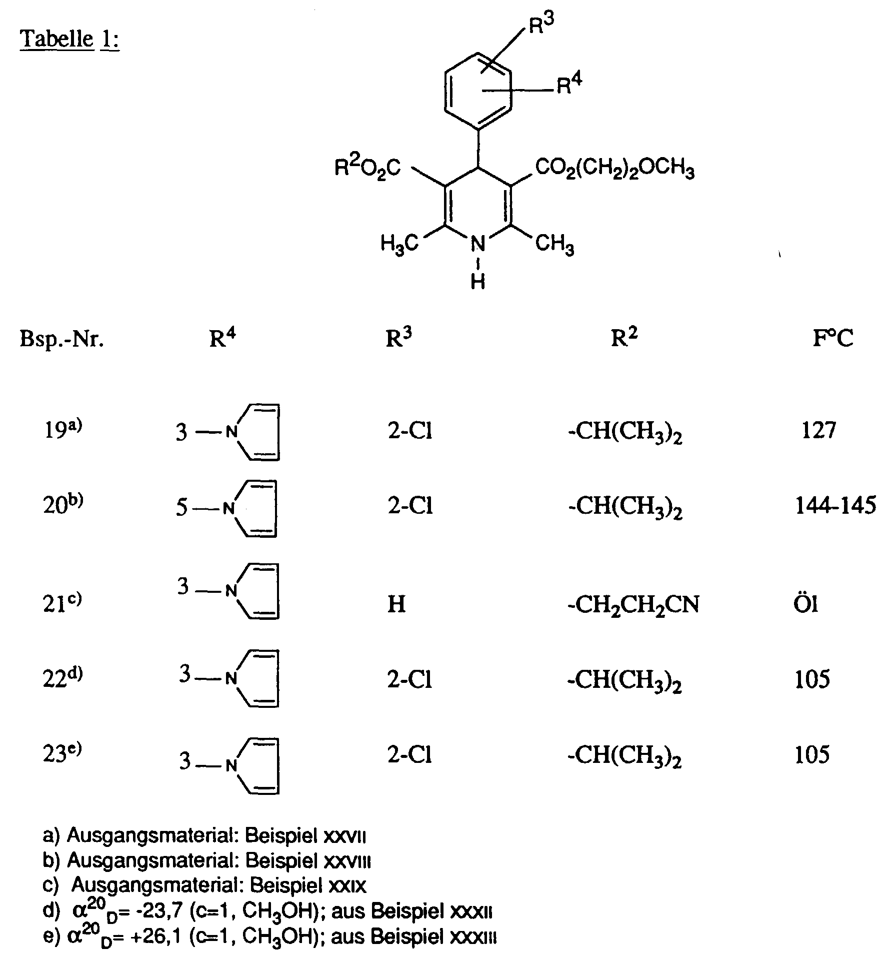

### Beispiel 24

1,4-Dihydro-2,6-dimethyl-4-[3-(2,5-dimethyl-pyrrol-1-yl)phenyl]-pyridin-3,5-dicarbonsäure-isopropyl-2-methoxyethylester

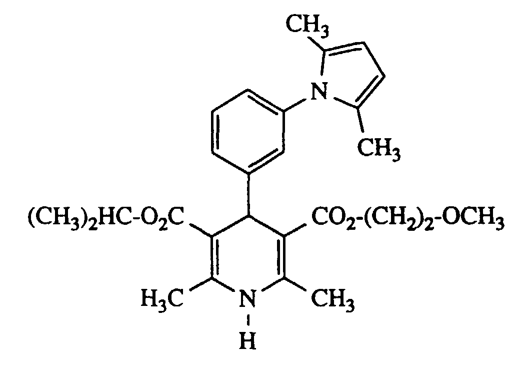

3,9 g (10 mmol) der Verbindung aus Beispiel XXX in 40 ml Toluol werden mit einer Spatelspitze p-Toluolsulfonsäure und 1,4 g (12 mmol) 2,5-Hexandion vesetzt. Nach 30 min bei 120°C wird über wenig Kieselgel filtriert und eingeengt. Das Rohprodukt wird durch Flashchromatographie (Toluol/Essigester 2:1) gereinigt. Das erhaltene Öl (2,3 g) wird in der Kälte mit Diethylether verrieben. Nach Zusatz von Cyclohexan werden die sich abscheidenden Kristalle abgetrennt.
Ausbeute: 0,52 g (11% d.Th.)
Schmp. 106-107°C

### Beispiel 25

4-[2-Chlor-5-(2,5-dimethyl-pyrrol-1-yl)phenyl]-pyridin-3,5-dicarbonsäure-isopropyl-2-methoxymethylester

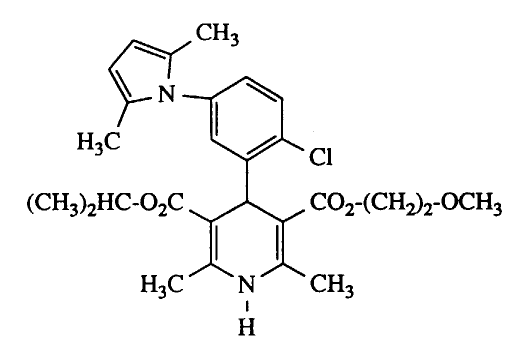

Die Titelverbindung wird in Analogie zu Beispiel 24 aus 3,6 g (8,6 mmol) der Verbindung aus Beispiel XXVIII hergestellt.
FP°C: 147-149°C

### Beispiel 26

1,4-Dihydro-2,6-dimethyl-4-[(3-pyrrol-1-yl)phenyl]-pyridin-3,5-dicarbonsäure-cyclopentyl-2-methoxyethylester

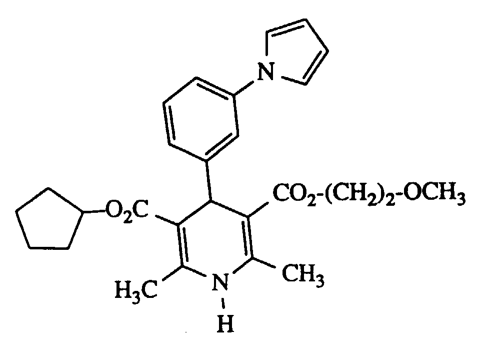

15,5 g (34,5 mmol) Verbindung aus Beispiel 21 in 150 ml Dimethoxyethan werden mit 70 ml 1M Natronlauge versetzt und 45 Minuten bei 50°C gerührt. Der auf RT abgekühlte Ansatz wird mehrmals mit Diethylether extrahiert. Die wäßrige Phase wird vorsichtig mit konzentrierter Salzsäure angesäuert. Das ausgefallene Rohprodukt wird über Phosphorpentoxid im Vakuum getrocknet. Umkristallisation aus 2-Propanol und 2-maliges Aufarbeiten der Mutterlaugen ergeben 7,1 g (54% d.Th.) 1,4-Dihydro-2,6-dimethyl-4-[(3-pyrrol-1-yl)phenyl]-pyridin-3,5-dicarbonsäure-2-methoxyethyl-monoester, die als Rohprodukt weiter umgesetzt werden.
3,0 g (7,6 mmol) hiervon in 30 ml Tetrahydrofuran werden mit 1,1 g (6,8 mmol) Carbonyldiimidazol versetzt und 45 Minuten bei 80°C gerührt. Nach Entfernen des Lösemittels im Vakuum wird in 10 ml Cyclopentanol aufgenommen und mit einer Spatelspitze 4-Dimethylaminopyridin versetzt. Das Gemisch wird 2 h bei 100°C gerührt. Chromatographie an Kieselgel mit Cyclohexan / Essigester 3:1 und anschließendes Verrühren des Rohprodukts liefert nach Aufarbeiten der Mutterlaugen 0,73 g (23% d.Th.) farblose Kristalle.
Schmp.: 124°C

## Patentansprüche

1. 4-Phenyl-dihydropyridine, die durch ungesättigten Heterocyclylrest substituiert sind, der allgemeinen Formel I 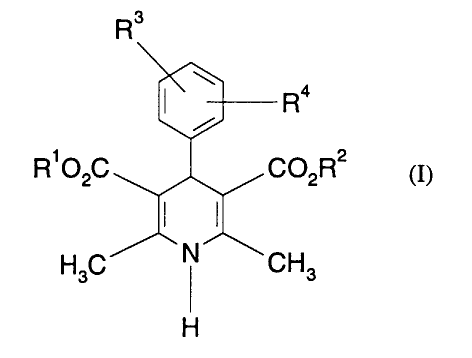 in welcher
R¹ und R² gleich oder verschieden sind und für Cycloalkyl mit 3 bis 8 Kohlenstoffatomen stehen, oder
für geradkettiges oder verzweigtes Alkyl mit bis zu 8 Kohlenstoffatomen stehen, das gegebenenfalls durch geradkettiges oder verzweigtes Alkoxy mit bis zu 6 Kohlenstoffatomen, Cyano, Cycloalkyl mit 3 bis 8 Kohlenstoffatomen oder Aryloxy mit 6 bis 10 Kohlenstoffatomen substituiert ist, das seinerseits bis zu 3-fach gleich oder verschieden durch Halogen, Cyano oder durch geradkettiges oder verzweigtes Alkyl oder Alkoxy mit bis zu 6 Kohlenstoffatomen substituiert sein kann,
R³ für Wasserstoff, Halogen, Trifluormethyl oder Cyano steht,
und
R⁴ für einen 5-gliedrigen ungesättigten, über ein Ring-Kohlenstoffatom angebundenen Heterocyclus mit bis zu 4 Heteroatomen aus der Reihe N und/oder O steht, der gegebenenfalls durch Hydroxy, geradkettiges oder verzweigtes Alkyl oder Alkoxycarbonyl mit jeweils bis zu 6 Kohlenstoffatomen, Trifluormethyl, Halogen, Carboxy oder durch eine Gruppe der Formel -NR⁵R⁶ substituiert ist,
oder
R⁴ einen Rest der Formel 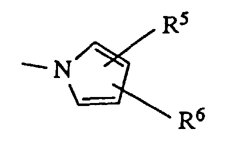 bedeutet,
worin
R⁵ und R⁶ gleich oder verschieden sind und Wasserstoff oder geradkettiges oder verzweigtes Alkyl oder Alkoxycarbonyl mit jeweils bis zu 4 Kohlenstoffatomen bedeuten,
und deren Salze,
mit der Maßgabe, daß wenn R¹ und R² für Ethyl und R³ für Wasserstoff stehen, R⁴ nicht den Rest der Formel 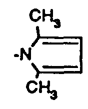 bedeuten darf.

2. 4-Heterocyclophenyl substituierte Dihydropyridine nach Anspruch 1,
worin
R¹ und R² gleich oder verschieden sind und für Cyclopropyl, Cyclopentyl, Cyclohexyl oder Cycloheptyl stehen, oder
für geradkettiges oder verzweigtes Alkyl mit bis zu 6 Kohlenstoffatomen stehen, das gegebenenfalls durch geradkettiges oder verzweigtes Alkoxy mit bis zu 4 Kohlenstoffatomen, Cyano, Cyclopropyl, Cyclopentyl, Cyclohexyl, Cycloheptyl oder Phenoxy substituiert ist, das seinerseits bis zu 3-fach gleich oder verschieden durch Fluor, Cyano oder durch geradkettiges oder verzweigtes Alkyl oder Alkoxy mit jeweils bis zu 4 Kohlenstoffatomen substituiert sein kann,
R³ für Wasserstoff, Fluor, Chlor, Brom, Trifluormethyl oder Cyano steht,
und
R⁴ für über ein Ring-Kohlenstoffatom gebundenes Tetrazolyl, 1,2,4-Oxadiazolyl oder 1,3,4-Oxadiazolyl steht, das gegebenenfalls durch Hydroxy, geradkettiges oder verzweigtes Alkyl oder Alkoxycarbonyl mit jeweils bis zu 4 Kohlenstoffatomen, Trifluormethyl, Fluor, Chlor oder durch eine Gruppe der Formel -NR⁵R⁶ substituiert ist,
oder
R⁴ einen Rest der Formel 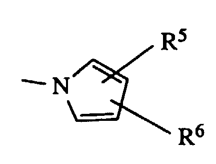 bedeutet,
worin
R⁵ und R⁶ gleich oder verschieden sind und Wasserstoff oder geradkettiges oder verzweigtes Alkyl oder Alkoxycarbonyl mit jeweils bis zu 3 Kohlenstoffatomen bedeuten.
und deren Salze,
mit der Maßgabe, daß wenn R¹ und R² für Ethyl und R³ für Wasserstoff stehen, R⁴ nicht den Rest der Formel 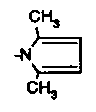 bedeuten darf.

3. 4-Heterocyclophenyl substituierte Dihydropyridine nach Anspruch 1,
worin
R¹ und R² gleich oder verschieden sind und für Cyclopentyl, Cyclohexyl oder Cycloheptyl stehen, oder
für geradkettiges oder verzweigtes Alkyl mit bis zu 4 Kohlenstoffatomen stehen, das gegebenenfalls durch Methoxy, Cyclopentyl, Cyclohexyl oder Cycloheptyl substituiert ist
R³ für Wasserstoff, Chlor, Trifluormethyl oder Cyano steht,
und
R⁴ für über ein Ring-Kohlenstoffatom gebundenes Tetrazolyl, 1,2,4-Oxadiazolyl oder 1,3,4-Oxadiazolyl steht, das gegebenenfalls durch geradkettiges oder verzweigtes Alkyl oder Alkoxycarbonyl mit jeweils bis zu 3 Kohlenstoffatomen, Trifluormethyl, Fluor, Chlor oder Amino substituiert ist,
R⁴ einen Rest der Formel 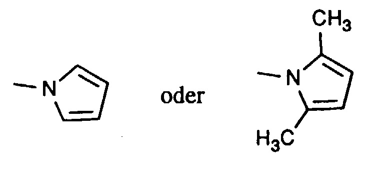 bedeutet.
und deren Salze,
mit der Maßgabe, daß wenn R¹ und R² für Ethyl und R³ für Wasserstoff stehen, R⁴ nicht den Rest der Formel 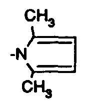 bedeuten darf.

4. 4-Tetrazolyl, 1,2,4 und 1,3,4-Oxadiazolylphenyl substituierte Dihydropyridine der Reihe:
1,4-Dihydro-2,6-dimethyl-4-[3-(1,2,4-oxadiazol-3-yl)phenyl]-pyridin-3,5-dicarbonsäure-isopropyl-2-methoxyethyl-ester
4-[3-(5-Ethoxycarbonyl-1,2,4-oxadiazol-3-yl)-phenyl]-1,4- dihydro-2,6-dimethyl-pyridin-3-5-dicarbonsäure-isopropyl-2-methoxyethyl-ester
(+)-4-[3-(5-Ethoxycarbonyl-1,2,4-oxadiazol-3-yl)-phenyl]-1,4-dihydro-2,6-dimethyl-pyridin-3-5-dicarbonsäure-isopropyl-2-methoxyethyl-ester
(-)-4-[3-(5-Ethoxycarbonyl-1,2,4-oxadiazol-3-yl)-phenyl]-1,4-dihydro-2,6-dimethyl-pyridin-3-5-dicarbonsäure-isopropyl-2-methoxyethyl-ester
1,4-Dihydro-2,6-dimethyl-4-[3-(tetrazol-5-yl)phenyl]-pyridin-3,5-dicarbonsäure-isopropyl-2-methoxyethyl-ester
1,4-Dihydro-2,6-dimethyl-4-[3-(1,3,4-oxadiazol-2-yl)-phenyl]-pyridin-3,5-dicarbonsäure-isopropyl-2-methoxyethyl-ester
(+)-1,4-Dihydro-2,6-dimethyl-4-[3-(2-methyl-1,3,4-oxadiazol-5-yl)phenyl]-pyridin-3,5-dicarbonsäure-isopropyl-2-methoxyethyl-ester
(-)-1,4-Dihydro-2,6-dimethyl-4-[3-(2-methyl-1,3,4-oxadiazol-5-yl)phenyl]-pyridin-3,5-dicarbonsäure-isopropyl-2-methoxyethyl-ester
(-)-1,4-Dihydro-2,6-dimethyl-4-[3-(1,3,4-oxadiazol-2-yl)phenyl]-pyridin-3,5-dicarbonsäure-isopropyl-2-methoxyethyl-ester
(+)-1,4-Dihydro-2,6-dimethyl-4-[3-(1,3,4-oxadiazol-2-yl)phenyl]-pyridin-3,5-dicarbonsäure-isopropyl-2-methoxyethyl-ester
1,4-Dihydro-2,6-dimethyl-4-[3-(1,3,4-oxadiazol-2-yl)phenyl]-pyridin-3,5-dicarbonsäure-isopropyl-2-propoxyethyl-ester
und
4-Pyrrolyl und 2,5-Dimethylpyrrolylphenyl substituierte Dihydropyridine der Reihe:
1,4-Dihydro-2,6-dimethyl-4-[3-(pyrrol-1-yl)phenyl]-pyridin-3,5-dicarbonsäure-isopropyl-2-methoxyethyl-ester
1,4-Dihydro-2,6-dimethyl-4-[3-(2,5-dimethyl-pyrrol-1-yl)phenyl]-pyridin-3,5-dicarbonsäure-isopropyl-2-methoxyethyl-ester
4-[2-Chlor-3-(pyrrol-1-yl)phenyl]-1,4-dihydro-2,6-dimethyl-pyridin-3,5-dicarbonsäure-isopropyl-2-methoxyethyl-ester
(+)-4-[2-Chlor-3-(pyrrol-1-yl)phenyl]-1,4-dihydro-2,6-dimethyl-pyridin-3,5-dicarbonsäure-isopropyl-2-methoxyethyl-ester
(-)-4-[2-Chlor-3-(pyrrol-1-yl)phenyl]-1,4-dihydro-2,6-dimethyl-pyridin-3,5-dicarbonsäure-isopropyl-2-methoxyethyl-ester
4-[2-Chlor-5-(pyrrol-1-yl)phenyl]-1,4-dihydro-2,6-dimethyl-pyridin-3,5-dicarbonsäure-isopropyl-2-methoxyethyl-ester
4-[2-Chlor-3-(2,5-dimethylpyrrol-1-yl)phenyl]-1,4-dihydro-2,6-dimethylpyridin-3,5-dicarbonsäure-isopropyl-2-methoxyethylester
4-[2-Chlor-5-(2,5-dimethylpyrrol-1-yl)phenyl]-1,4-dihydro-2,6-dimethylpyridin-3,5-dicarbonsäure-isopropyl-2-methoxyethyl-ester
4-[2-Chlor-5-(2,5-dimethylpyrrol-1-yl)phenyl]-1,4-dihydro-2,6-dimethylpyridin-3,5-dicarbonsäure-isopropyl-cyclopentyl-ester.

5. 4-Heterocyclophenyl substituierte Dihydropyridine nach Anspruch 1 bis 4 zur therapeutischen Verwendung.

6. Verfahren zur Herstellung von 4-Heterocyclophenyl substituierten Dihydropyridinen nach Anspruch 1 bis 4, dadurch gekennzeichnet, daß man
[A] Aldehyde der allgemeinen Formel (II) 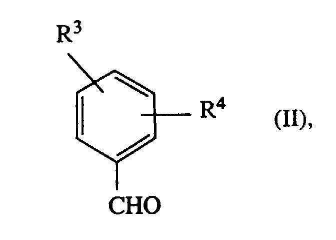 in welcher
R³ und R⁴ die oben angegebene Bedeutung haben,
zunächst mit Acetessigsäureestern der allgemeinen Formel (III)
H₃C-CO-CH₂-CO₂R² (III),
in welcher
R² die oben angegebene Bedeutung hat,
gegebenenfalls unter Isolierung der Benzylidenverbindung umsetzt,
und anschließend mit 2-Aminocrotonsäureestern der allgmeinen Formel (IV) 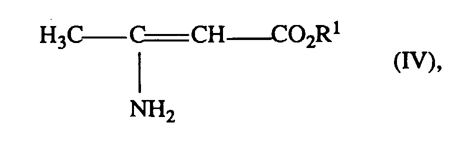 in welcher
R¹ die oben angegebene Bedeutung hat
in inerten Lösemitteln in Anwesenheit einer Base und gegebenenfalls Säuren umsetzt,
oder
[B] im Fall, daß der Substituent R⁴ für ein 1,3,4-Oxadiazolylrest steht,
Verbindungen der allgemeinen Formel (V) 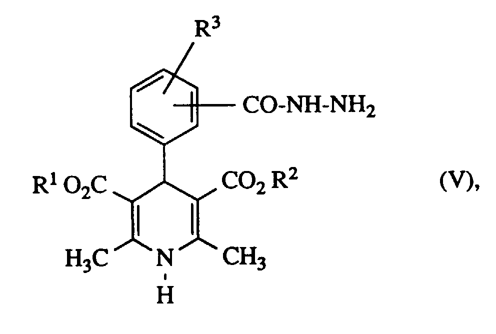 in welcher
R¹, R²und R³ die oben angegebene Bedeutung haben,
mit 1,1,1-Trialkoxyalkanen, und im Fall eines aminosubstituierten Oxadiazolylrestes mit Bromcyan oder Chlorcyan in Gegenwart von Basen umsetzt,
oder
[C] im Fall, daß R⁴ für ein 1,2,4-Oxadiazolylrest steht,
Verbindungen der allgemeinen Formel (VI) 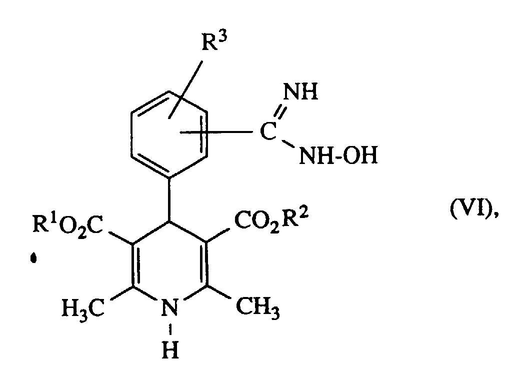 in welcher
R¹, R² und R³ die oben angegebene Bedeutung haben,
mit 1,1,1-Trialkoxyalkanen, Carbonsäurechloriden oder Carbonsäureanhydriden, in inerten Lösemitteln, gegebenenfalls in Anwesenheit von Basen, umsetzt,
oder
[D] im Fall, daß R⁴ für den Tetrazolylrest steht,
Verbindungen der allgemeinen Formel (VII) 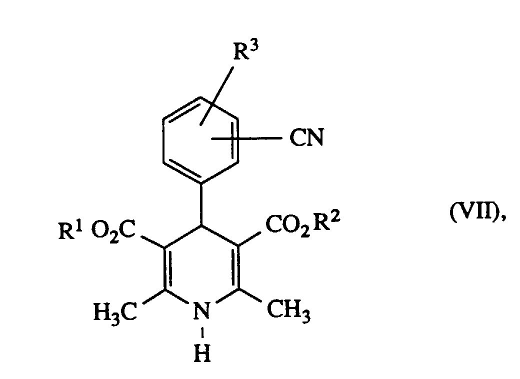 in welcher
R¹, R² und R³ die oben angegebene Bedeutung haben,
in inerten Lösemitteln, mit Natriumazid in Anwesenheit von Ammoniumchlorid umsetzt,
[E] im Fall, daß R⁴ einen über das Stickstoffatom gebundenen Pyrrolrest bedeutet
Verbindung der allgemeinen Formel (VIII) 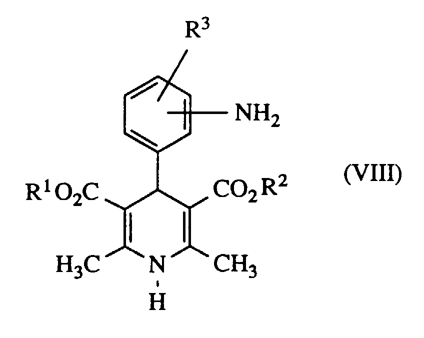 in welcher
R¹, R² und R³ die oben angegebene Bedeutung haben,
in inerten Lösemitteln mit 1,4-Dicarbonylverbindungen der allgemeinen Formel (IX) oder Di(C₁-C₄)alkoxyfuranen der allgemeinen Formel (X) 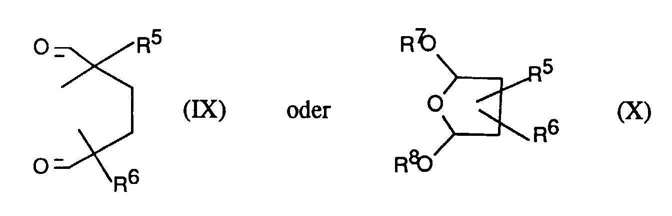 in welcher
R⁵ und R⁶ die oben angegebene Bedeutung haben
und
R⁷ und R⁸ gleich oder verschieden sind und C₁-C₄-Alkyl bedeuten,
in Gegenwart von Säuren umsetzt,
oder
[F] Verbindungen der allgemeinen Formel (XI) 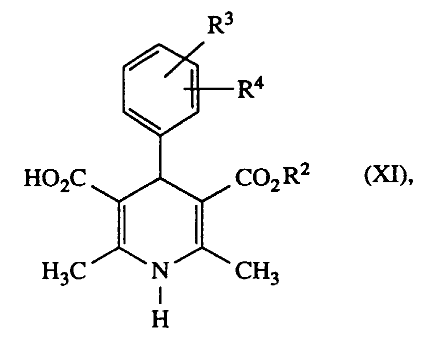 in welcher
R², R³ und R⁴ die oben angegebene Bedeutung haben,
gegebenenfalls über ein reaktives Säurederivat, in Anwesenheit einer Base, mit den entsprechenden Alkoholen umsetzt, wobei durch Einsatz der enantiomerenreinen Derivate der Verbindungen der allgemeinen Formel (X) die entsprechenden Enantiomeren der allgemeinen Formel (I) erhalten werden.

7. Arzneimittel enthaltend mindestens ein 4-Heterocyclophenyl substituiertes Dihydropyridin nach Ansprüchen 1 bis 4.

8. Arzneimittel nach Anspruch 7 zur Behandlung von cerebralen Erkrankungen.

9. Verfahren zur Herstellung von Arzneimitteln nach Anspruch 7, dadurch gekennzeichnet, daß man die 4-Heterocyclophenyl substituierten Dihydropyridine gegebenenfalls mit geeigneten Hilfs- und Trägerstoffen in eine geeignete Applikationsform überführt.

10. Verwendung von 4-Heterocyclophenyl substituierten Dihydropyridinen nach Anspruch 1 bis 4 zur Herstellung von Arzneimitteln.

## Claims

1. 4-Phenyl dihydropyridines, which are substituted by an unsaturated heterocyclyl radical, of the general formula I 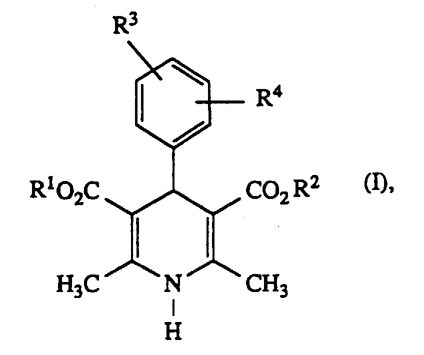 in which
R¹ and R² are identical or different and represent cycloalkyl having 3 to 8 carbon atoms, or
represent straight-chain or branched alkyl having up to 8 carbon atoms, which is optionally substituted by straight-chain or branched alkoxy having up to 6 carbon atoms, cyano, cycloalkyl having 3 to 8 carbon atoms or aryloxy having 6 to 10 carbon atoms, which can in turn be mono-, di- or trisubstituted by identical or different substituents from the group comprising halogen, cyano and straight-chain or branched alkyl or alkoxy having up to 6 carbon atoms,
R³ represents hydrogen, halogen, trifluoromethyl or cyano,
and
R⁴ represents a 5-membered unsaturated heterocyclic radical having up to 4 hetero atoms from the series comprising N and/or 0, which is bonded via a ring carbon atom and is optionally substituted by hydroxyl, straight-chain or branched alkyl or alkoxycarbonyl having in each case up to 6 carbon atoms, trifluoromethyl, halogen, carboxyl or a group of the formula -NR⁵R⁶,
or
R⁴ denotes a radical of the formula 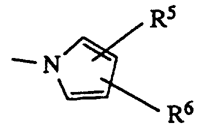 wherein
R⁵ and R⁶ are identical or different and denote hydrogen or straight-chain or branched alkyl or alkoxycarbonyl having in each case up to 4 carbon atoms,
and salts thereof,
with the proviso that if R¹ and R² represent ethyl and R³ represents hydrogen, R⁴ may not denote the radical of the formula

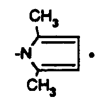

2. 4-Heterocyclophenyl-substituted dihydropyridines according to Claim 1,
wherein
R¹ and R² are identical or different and represent cyclopropyl, cyclopentyl, cyclohexyl or cycloheptyl, or
represent straight-chain or branched alkyl having up to 6 carbon atoms, which is optionally substituted by straight-chain or branched alkoxy having up to 4 carbon atoms, cyano, cyclopropyl, cyclopentyl, cyclohexyl, cycloheptyl or phenoxy, which can in turn be mono-, di- or trisubstituted by identical or different substituents from the group comprising fluorine, cyano and straight-chain or branched alkyl or alkoxy having in each case up to 4 carbon atoms,
R³ represents hydrogen, fluorine, chlorine, bromine, trifluoromethyl or cyano,
and
R⁴ represents a tetrazolyl, 1,2,4-oxadiazolyl or 1,3,4-oxadiazolyl radical which is bonded via a ring carbon atom and is optionally substituted by hydroxyl, straight-chain or branched alkyl or alkoxycarbonyl having in each case up to 4 carbon atoms, trifluoromethyl, fluorine, chlorine or a group of the formula -NR⁵R⁶,
or
R⁴ denotes a radical of the formula 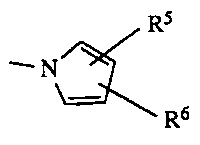 wherein
R⁵ and R⁶ are identical or different and denote hydrogen or straight-chain or branched alkyl or alkoxycarbonyl having in each case up to 3 carbon atoms,
and salts thereof,
with the proviso that if R¹ and R² represent ethyl and R³ represents hydrogen, R⁴ may not denote the radical of the formula

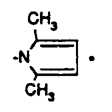

3. 4-Heterocyclophenyl-substituted dihydropyridines according to Claim 1,
wherein
R¹ and R² are identical or different and represent cyclopentyl, cyclohexyl or cycloheptyl, or represent straight-chain or branched alkyl having up to 4 carbon atoms, which is optionally substituted by methoxy, cyclopentyl, cyclohexyl or cycloheptyl,
R³ represents hydrogen, chlorine, trifluoromethyl or cyano,
and
R⁴ represents a tetrazolyl, 1,2,4-oxadiazolyl or 1,3,4-oxadiazolyl radical which is bonded via a ring carbon atom and is optionally substituted by straight-chain or branched alkyl or alkoxycarbonyl having in each case up to 3 carbon atoms, trifluoromethyl, fluorine, chlorine or amino,
R⁴ denotes a radical of the formula 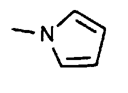 or 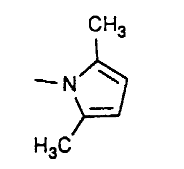 and salts thereof,
with the proviso that if R¹ and R² represent ethyl and R³ represents hydrogen, R⁴ may not denote the radical of the formula

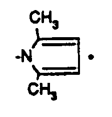

4. 4-Tetrazolyl- and 1,2,4- and 1,3,4-oxadiazolylphenyl-substituted dihydropyridines of the series comprising:
Isopropyl 2-methoxyethyl 1,4-dihydro-2,6-dimethyl-4-[3-(1,2,4-oxadiazol-3-yl)phenyl]-pyridine-3,5-dicarboxylate
Isopropyl 2-methoxyethyl 4-[3-(5-ethoxycarbonyl-1,2,4-oxadiazol-3-yl)-phenyl]-1,4-dihydro-2,6-dimethyl-pyridine-3,5-dicarboxylate
Isopropyl 2-methoxyethyl (+)-4-[3-(5-ethoxycarbonyl-1,2,4-oxadiazol-3-yl)-phenyl]-1,4-dihydro-2,6-dimethyl-pyridine-3,5-dicarboxylate
Isopropyl 2-methoxyethyl (-)-4-[3-(5-ethoxycarbonyl-1,2,4-oxadiazol-3-yl)-phenyl]-1,4-dihydro-2,6-dimethyl-pyridine-3,5-dicarboxylate
Isopropyl 2-methoxyethyl 1,4-dihydro-2,6-dimethyl-4-[3-(tetrazol-5-yl)phenyl]-pyridine-3,5-dicarboxylate
Isopropyl 2-methoxyethyl 1,4-dihydro-2,6-dimethyl-4-[3-(1,3,4-oxadiazol-2-yl)-phenyl]pyridine-3,5-dicarboxylate
Isopropyl 2-methoxyethyl (+)-1,4-dihydro-2,6-dimethyl-4-[3-(2-methyl-1,3,4-oxadiazol-5-yl)-phenyl]-pyridine-3,5-dicarboxylate
Isopropyl 2-methoxyethyl (-)-1,4-dihydro-2,6-dimethyl-4-[3-(2-methyl-1,3,4-oxadiazol-5-yl)-phenyl]-pyridine-3,5-dicarboxylate
Isopropyl 2-methoxyethyl (-)-1,4-dihydro-2,6-dimethyl-4-[3-(1,3,4-oxadiazol-2-yl)-phenyl]pyridine-3,5-dicarboxylate
Isopropyl 2-methoxyethyl (+)-1,4-dihydro-2,6-dimethyl-4-[3-(1,3,4-oxadiazol-2-yl)-phenyl]pyridine-3,5-dicarboxylate and
Isopropyl 2-propoxyethyl 1,4-dihydro-2,6-dimethyl-4-[3-(1,3,4-oxadiazol-2-yl)phenyl]-pyridine-3,5-dicarboxylate
and
4-pyrrolyl- and 2,5-dimethylpyrrolylphenyl-substituted dihydropyridines of the series comprising:
Isopropyl 2-methoxyethyl 1,4-dihydro-2,6-dimethyl-4-[3-(pyrrol-1-yl)phenyl]-pyridine-3,5-dicarboxylate
Isopropyl-2-methoxyethyl 1,4-dihydro-2,6-dimethyl-4-[3-(2,5-dimethyl-pyrrol-1-yl)phenyl]-pyridine-3,5-dicarboxylate,
Isopropyl 2-methoxyethyl 4-[2-chloro-3-(pyrrol-1-yl)phenyl]-1,4-dihydro-2,6-dimethyl-pyridine-3,5-dicarboxylate
Isopropyl 2-methoxyethyl (+)-4-[2-chloro-3-(pyrrol-1-yl)phenyl]-1,4-dihydro-2,6-dimethyl-pyridine-3,5-dicarboxylate
Isopropyl 2-methoxyethyl (-)-4-[2-chloro-3-(pyrrol-1-yl)phenyl]-1,4-dihydro-2,6-dimethyl-pyridine-3,5-dicarboxylate
Isopropyl 2-methoxyethyl 4-[2-chloro-5-(pyrrol-1-yl)phenyl]-1,4-dihydro-2,6-dimethyl-pyridine-3,5-dicarboxylate
Isopropyl 2-methoxyethyl 4-[2-chloro-3-(2,5-dimethylpyrrol-1-yl)phenyl]-1,4-dihydro-2,6-dimethylpyridine-3,5-dicarboxylate
Isopropyl 2-methoxyethyl 4-[2-chloro-5-(2,5-dimethylpyrrol-1-yl)phenyl]-1,4-dihydro-2,6-dimethylpyridine-3,5-dicarboxylate and
Isopropyl cyclopentyl 4-[2-chloro-5-(2,5-dimethylpyrrol-1-yl)phenyl]-1,4-dihydro-2,6-dimethyl-pyridine-3,5-dicarboxylate.

5. 4-Heterocyclophenyl-substituted dihydropyridines according to Claim 1 to 4 for therapeutic use.

6. Process for the preparation of 4-heterocyclophenyl-substituted dihydropyridines according to Claim 1 to 4, characterized in that
[A] aldehydes of the general formula (II) 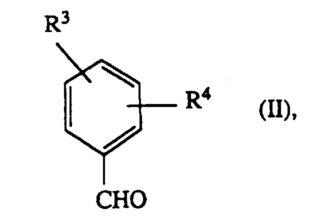 in which
R³ and R⁴ have the abovementioned meaning,
are first reacted with acetoacetic acid esters of the general formula (III)
H₃C-CO-CH₂-CO₂R² (III),
in which
R² has the abovementioned meaning,
if appropriate with isolation of the benzylidene compound,
and the products are then reacted with 2-aminocrotonic acid esters of the general formula (IV) 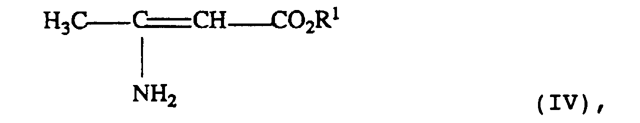 in which
R¹ has the abovementioned meaning,
in inert solvents in the presence of a base and if appropriate acids,
or
[B] in the case where the substituent R⁴ represents a 1,3,4-oxadiazolyl radical,
compounds of the general formula (V) 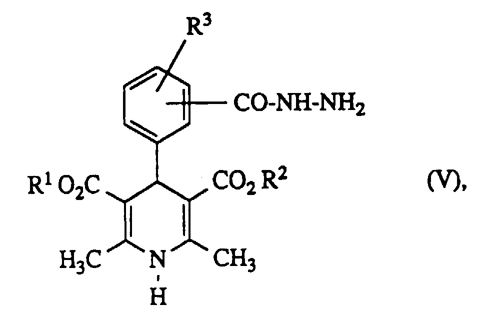 in which
R¹, R² and R³ have the abovementioned meaning,
are reacted with 1,1,1-trialkoxyalkanes, and in the case of an amino-substituted oxadiazolyl radical, the products are reacted with cyanogen bromide or cyanogen chloride in the presence of bases,
or
[C] in the case where R⁴ represents a 1,2,4-oxadiazolyl radical,
compounds of the general formula (VI) 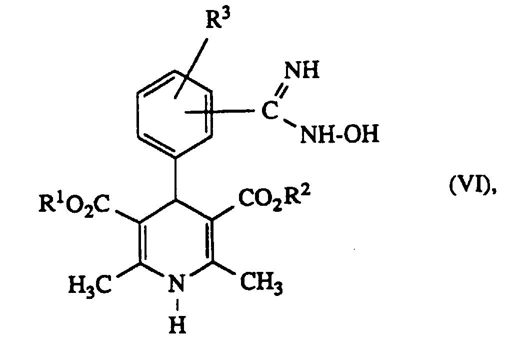 in which
R¹, R² and R³ have the abovementioned meaning,
are reacted with 1,1,1-trialkoxyalkanes, carboxylic acid chlorides or carboxylic acid anhydrides in inert solvents, if appropriate in the presence of bases,
or
[D] in the case where R⁴ represents the tetrazolyl radical,
compounds of the general formula (VII) 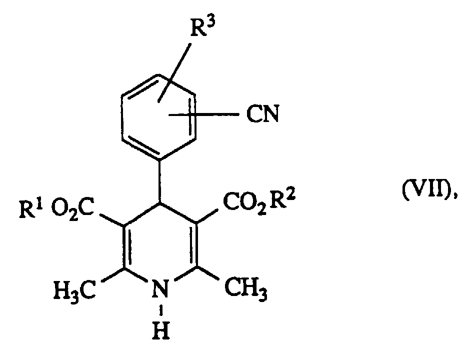 in which
R¹, R² and R³ have the abovementioned meaning,
are reacted with sodium azide in inert solvents in the presence of ammonium chloride,
or
[E] in the case where R⁴ denotes a pyrrole radical bonded via the nitrogen atom,
compound of the general formula (VIII) 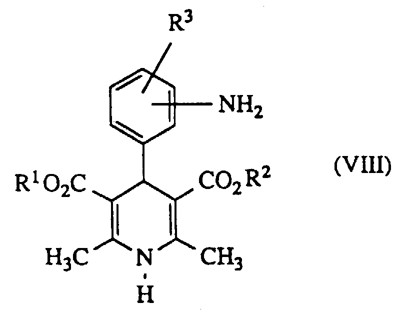 in which
R¹, R² and R³ have the abovementioned meaning,
is reacted with 1,4-dicarbonyl compounds of the general formula (IX) or di(C₁-C₄)alkoxyfurans of the general formula (X) 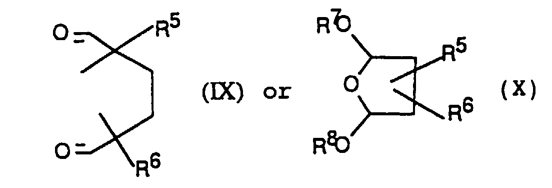 in which
R⁵ and R⁶ have the abovementioned meaning
and
R⁷ and R⁸ are identical or different and denote C₁-C₄-alkyl,
in inert solvents in the presence of acids,
or
[F] compounds of the general formula (XI) 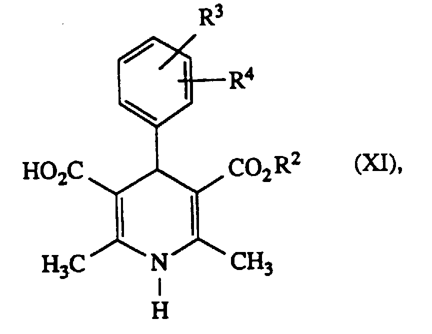 in which
R², R³ and R⁴ have the abovementioned meaning,
are reacted with the corresponding alcohols, if appropriate via a reactive acid derivative, in the presence of a base, wherein by using the enantiomerically pure derivatives of the compounds of the general formula (X), the corresponding enantiomers of the general formula (I) are obtained.

7. Medicaments comprising at least one 4-heterocyclophenyl-substituted dihydropyridine according to Claims 1 to 4.

8. Medicaments according to Claim 7 for the treatment of cerebral diseases.

9. Process for the preparation of medicaments according to Claim 7, characterized in that the 4-heterocyclophenyl-substituted dihydropyridines are converted into a suitable administration form, if appropriate with suitable auxiliaries and excipients.

10. Use of 4-heterocyclophenyl-substituted dihydropyridines according to Claim 1 to 4 for the preparation of medicaments.

## Revendications

1. 4-phényl-dihydropyridines, qui sont substituées par un reste hétérocyclyle non saturé, de formule générale I 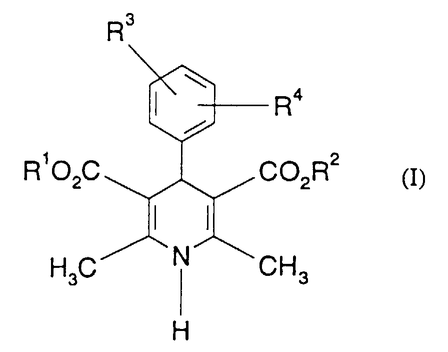 dans laquelle
R¹ et R² sont identiques ou différents et représentent un groupe cycloalkyle ayant 3 à 8 atomes de carbone, ou
un groupe alkyle linéaire ou ramifié ayant jusqu'à 8 atomes de carbone, qui est substitué, le cas échéant, par un radical alkoxy linéaire ou ramifié ayant jusqu'à 6 atomes de carbone, cyano, cycloalkyle ayant 3 à 8 atomes de carbone ou aryloxy ayant 6 à 10 atomes de carbone, qui peut lui-même être substitué, jusqu'à trois fois identiques ou différentes, par un radical halogéno, cyano ou par un radical alkyle ou alkoxy linéaire ou ramifié ayant jusqu'à 6 atomes de carbone,
R³ représente de l'hydrogène, un halogène, un groupe trifluorométhyle ou cyano,
et
R⁴ est un hétérocycle pentagonal non saturé, lié par un atome de carbone du noyau, ayant jusqu'à 4 hétéroatomes de la série N et/ou O, qui est substitué, le cas échéant, par un radical hydroxy, alkyle ou alkoxycarbonyle linéaire ou ramifié ayant chacun jusqu'à 6 atomes de carbone, trifluorométhyle, halogéno, carboxy ou par un groupe de formule -NR⁵R⁶,
ou
R⁴ est un reste de formule 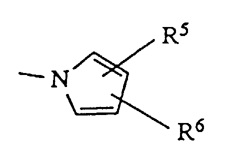 dans laquelle
R⁵ et R⁶ sont identiques ou différents et représentent de l'hydrogène ou un groupe alkyle ou un groupe alkoxycarbonyle linéaire ou ramifié ayant chacun jusqu'à 4 atomes de carbone,
et leurs sels,
sous réserve que lorsque R¹ et R² représentent un groupe éthyle et R³ est de l'hydrogène, R⁴ ne puisse pas représenter le reste de formule

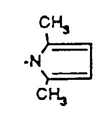

2. Dihydropyridines à substituant 4-hétérocyclophényle suivant la revendication 1, dans lesquelles
R¹ et R² sont identiques ou différents et représentent un groupe cyclopropyle, cyclopentyle, cyclohexyle ou cycloheptyle, ou bien
un groupe alkyle linéaire ou ramifié ayant jusqu'à 6 atomes de carbone, qui est substitué, le cas échéant, par un radical alkoxy linéaire ou ramifié ayant jusqu'à 4 atomes de carbone, cyano, cyclopropyle, cyclopentyle, cyclohexyle, cycloheptyle ou phénoxy, qui peut lui-même être substitué, jusqu'à trois fois identiques ou différentes, par du fluor, un radical cyano ou par un radical alkyle ou un radical alkoxy linéaire ou ramifié ayant chacun jusqu'à 4 atomes de carbone,
R³ est de l'hydrogène, du fluor, du chlore, du brome, un groupe trifluorométhyle ou cyano,
et
R⁴ représente un groupe tétrazolyle, 1,2,4-oxadiazolyle ou 1,3,4-oxoadiazolyle lié par un atome de carbone du noyau, qui est substitué, le cas échéant, par un radical hydroxy, alkyle ou alkoxycarbonyle linéaire ou ramifié ayant chacun jusqu'à 4 atomes de carbone, trifluorométhyle, fluoro, chloro ou par un groupe de formule -NR⁵R⁶,
ou bien
R⁴ représente un reste de formule 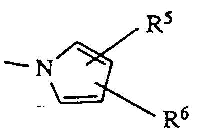 dans laquelle
R⁵ et R⁶ sont identiques ou différents et représentent de l'hydrogène ou un groupe alkyle ou un groupe alkoxycarbonyle linéaire ou ramifié ayant chacun jusqu'à 3 atomes de carbone,
et leur sels,
sous réserve que lorsque R¹ et R² représentent un groupe éthyle et R³ est de l'hydrogène, R⁴ ne puisse pas représenter le reste de formule

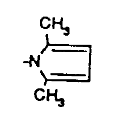

3. Dihydropyridines à substituant 4-hétérocyclophényle suivant la revendication 1, dans lesquelles
R¹ et R² sont identiques ou différents et représentent un groupe cyclopentyle, cyclohexyle ou cycloheptyle, ou bien
un groupe alkyle linéaire ou ramifié ayant jusqu'à 4 atomes de carbone, qui est substitué, le cas échéant, par un radical méthoxy, cyclopentyle, cyclohexyle ou cycloheptyle,
R³ est de l'hydrogène, du chlore, un groupe trifluorométhyle ou cyano,
et
R⁴ représente un groupe tétrazolyle, 1,2,4-oxadiazolyle ou 1,3,4-oxadiazolyle lié par un atome de carbone du noyau, qui est substitué, le cas échéant, par un radical alkyle ou un radical alkoxycarbonyle linéaire ou ramifié ayant chacun jusqu'à 3 atomes de carbone, trifluorométhyle, fluoro, chloro ou amino,
R⁴ est un reste de formule 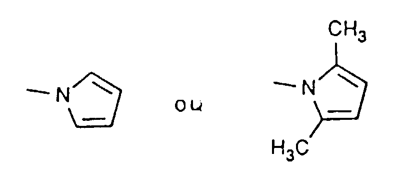
et leurs sels,
sous réserve que, lorsque R¹ et R² représentent un groupe éthyle et R³ est de l'hydrogène, R⁴ ne puisse pas représenter le reste de formule

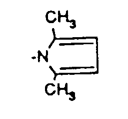

4. Dihydropyridines à substituants 4-tétrazolyle, 1,2,4- et 1,3,4-oxadiazolylphényle, de la série :
ester d'isopropyle et de 2-méthoxyéthyle de l'acide 1,4-dihydro-2,6-diméthyl-4-[3-(1,2,4-oxadiazole-3-yl)phényl]pyridine-3,5-dicarboxylique,
ester d'isopropyle et de 2-méthoxyéthyle d'acide 4-[3-(5-éthoxycarbonyl-1,2,4-oxadiazole-3-yl)phényl]-1,4-dihydro-2, 6-diméthylpyridine-3,5-dicarboxylique,
ester d'isopropyle et de 2-méthoxyéthyle d'acide (+)-4-[3-(5-ethoxycarbonyl-1,2,4-oxadiazole-3-yl)phényl]-1,4-dihydro-2,6-diméthylpyridine-3,5-dicarboxylique,
ester d'isopropyle et de 2-méthoxyéthyle d'acide (-)-4-[3-(5-éthoxycarbonyl-1,2,4-oxadiazole-3-yl)phényl]-1,4-dihydro-2,6-diméthylpyridine-3,5-dicarboxylique,
ester d'isopropyle et de 2-méthoxyéthyle d'acide 1,4-dihydro-2,6-diméthyl-4-[3-(tétrazole-5-yl)phényl]-pyridine-3,5-dicarboxylique,
ester d'isopropyle et de 2-méthoxyéthyle d'acide 1,4-dihydro-2,6-diméthyl-4-[3-(1,3,4-oxadiazole-2-yl)phényl]-pyridine-3,5-dicarboxylique,
ester d'isopropyle et de 2-méthoxyéthyle d'acide (+)-1,4-dihydro-2,6-diméthyl-4-[3-(2-méthyl-1,3,4-oxadiazole-5-yl)phényl]pyridine-3,5-dicarboxylique,
ester d'isopropyle et de 2-méthoxyéthyle d'acide (-)-1,4-dihydro-2,6-diméthyl-4-[3-(2-méthyl-1,3,4-oxadiazole-5-yl)phényl]pyridine-3,5-dicarboxylique,
ester d'isopropyle et de 2-méthoxyéthyle d'acide (-)-1,4-dihydro-2,6-diméthyl-4-[3-(1,3,4-oxadiazole-2-yl)phényl]pyridine-3,5-dicarboxylique,
ester d'isopropyle et de 2-méthoxyéthyle d'acide (+)-1,4-dihydro-2,6-diméthyl-4-[3-(1,3,4-oxadiazole-2-yl)phényl]pyridine-3,5-dicarboxylique,
ester d'isopropyle et de 2-propoxyéthyle d'acide 1,4-dihydro-2,6-diméthyl-4-[3-(1,3,4-oxadiazole-2-yl)phényl]-pyridine-3,5-dicarboxylique,
et
dihydropyridines à substituants 4-pyrrolyle et 2,5-diméthylpyrrolylphényle de la série :
ester d'isopropyle et de 2-méthoxyéthyle d'acide 1,4-dihydro-2,6-diméthyl-4-[3-(pyrrole-1-yl)phényl]pyridine-3,5-dicarboxylique,
ester d'isopropyle et de 2-méthoxyéthyle d'acide 1,4-dihydro-2,6-diméthyl-4-[3-(2,5-diméthylpyrrole-1-yl)-phényl]pyridine-3,5-dicarboxylique,
ester d'isopropyle et de 2-méthoxyéthyle d'acide 4-[2-chloro-3-(pyrrole-1-yl)phényl]-1,4-dihydro-2,6-diméthylpyridine-3,5-dicarboxylique,
ester d'isopropyle et de 2-méthoxyéthyle d'acide (+)-4-[2-chloro-3-(pyrrole-1-yl)phényl]-1,4-dihydro-2,6-diméthylpyridine-3,5-dicarboxylique,
ester d'isopropyle et de 2-méthoxyéthyle d'acide (-)-4-[2-chloro-3-(pyrrole-1-yl)phényl]-1,4-dihydro-2,6-diméthylpyridine-3,5-dicarboxylique,
ester d'isopropyle et de 2-méthoxyéthyle d'acide 4-[2-chloro-5-(pyrrole-1-yl)phényl]-1,4-dihydro-2,6-diméthylpyridine-3,5-dicarboxylique,
ester d'isopropyle et de 2-méthoxyéthyle d'acide 4-[2-chloro-3-(2,5-diméthylpyrrole-1-yl)phényl]-1,4-dihydro-2,6-diméthylpyridine-3,5-dicarboxylique,
ester d'isopropyle et de 2-méthoxyéthyle d'acide 4-[2-chloro-5-(2,5-diméthylpyrrole-1-yl)phényl]-1,4-dihydro-2,6-diméthylpyridine-3,5-dicarboxylique,
ester d'isopropyle et de cyclopentyle d'acide 4-[2-chloro-5-(2,5-diméthylpyrrole-1-yl)phényl]-1,4-dihydro-2,6-diméthylpyridine-3,5-dicarboxylique.

5. Dihydropyridines à substituant 4-hétérocyclophényle suivant les revendications 1 à 4, destinées à une utilisation thérapeutique.

6. Procédé de production de dihydropyridines à substituant 4-hétérocyclophényle suivant les revendications 1 à 4, caractérisé en ce que :
[A] on fait réagir des aldéhydes de formule générale (II) 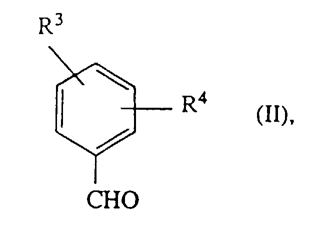 dans laquelle
R³ et R⁴ ont la définition indiquée ci-dessus,
tout d'abord avec des esters d'acide acétylacétique de formule générale (III)
H₃C-CO-CH₂-CO₂R² (III),
dans laquelle
R² a la définition indiquée ci-dessus,
le cas échéant avec isolement du composé de benzylidène,
puis on conduit la réaction avec des esters d'acide 2-aminocrotonique de formule générale (IV) 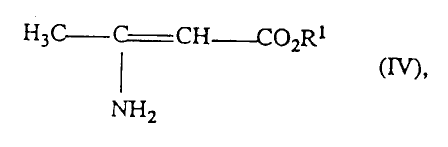 dans laquelle
R¹ a la définition indiquée ci-dessus,
dans des solvants inertes en présence d'une base et, le cas échéant, d'acides,
ou bien
[B] au cas où le substituant R⁴ représente un reste 1,3,4-oxadiazolyle,
on fait réagir des composés de formule générale (V) 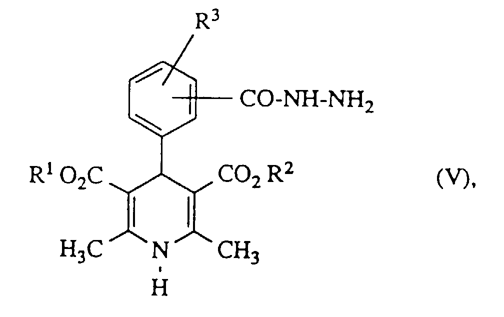 dans laquelle
R¹, R² et R³ ont la définition indiquée ci-dessus,
avec des 1,1,1-trialkoxyalcanes, et dans le cas d'un reste oxadiazolyle à substituant amino, avec le bromure de cyanogène ou le chlorure de cyanogène en présence de bases, ou bien
[C] au cas où R⁴ représente un reste 1,2,4-oxadiazolyle,
on fait réagir des composés de formule générale (VI) 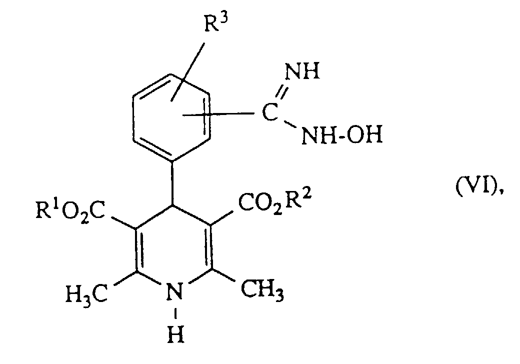 dans laquelle
R¹, R² et R³ ont la définition indiquée ci-dessus,
avec des 1,1,1-trialkoxyalcanes, des chlorures d'acides carboxyliques ou des anhydrides d'acides carboxyliques, dans des solvants inertes, le cas échéant en présence de bases,
ou bien
[D] au cas où R⁴ représente le reste tétrazolyle,
on fait réagir des composés de formule générale (VII) 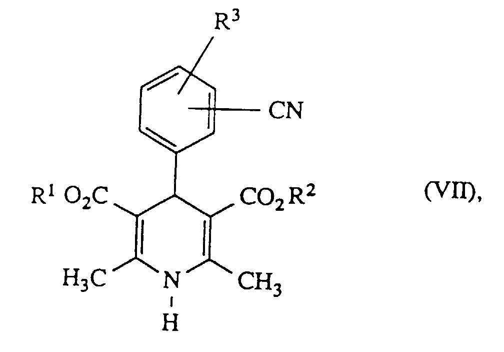 dans laquelle
R¹, R² et R³ ont la définition indiquée ci-dessus, dans des solvants inertes, avec l'azoture de sodium en présence de chlorure d'ammonium,
[E] au cas où R⁴ représente un reste pyrrole lié par l'intermédiaire de l'atome d'azote,
on fait réagir un composé de formule générale (VIII) 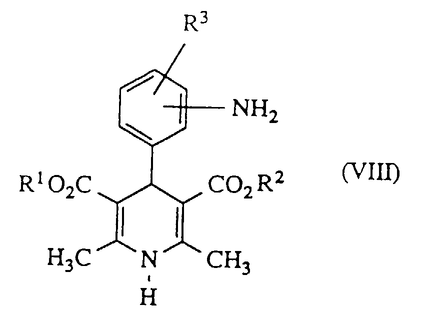 dans laquelle
R¹, R² et R³ ont la définition indiquée ci-dessus,
dans des solvants inertes, avec des composés 1,4-dicarbonyliques de formule générale (IX) ou avec des di-(alkoxy en C₁ à C₄)-furannes de formule générale (X) 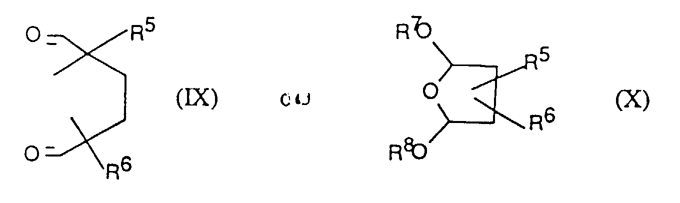 dans laquelle
R⁵ et R⁶ ont la définition indiquée ci-dessus
et
R⁷ et R⁸ sont identiques ou différents et représentent un groupe alkyle en C₁ à C₄,
en présence d'acides,
ou bien
[F] on fait réagir des composés de formule générale (XI) 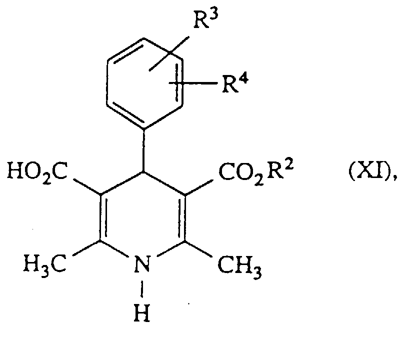 dans laquelle
R², R³ et R⁴ ont la définition indiquée ci-dessus,
le cas échéant, par l'intermédiaire d'un dérivé d'acide réactif, en présence d'une base, avec les alcools correspondants, et en utilisant les dérivés de pureté énantiomérique des composés de formule générale (X), on obtient alors les énantiomères correspondants de formule générale (I).

7. Médicament contenant une dihydropyridine à substituant 4-hétérocyclophényle suivant les revendications 1 à 4.

8. Médicament suivant la revendication 7, destiné au traitement de maladies cérébrales.

9. Procédé de préparation de médicaments suivant la revendication 7, caractérisé en ce qu'on fait prendre une forme d'administration appropriée aux dihydropyridines à substituant 4-hétérocyclophényle, le cas échéant avec des substituants auxiliaires et des supports appropriés.

10. Utilisation de dihydropyridines à substituant 4-hétérocyclophényle suivant les revendications 1 à 4 pour la préparation de médicaments.
